# EUROPEAN PATENT SPECIFICATION

(11) **EP 3 423 598 B1**
(45) Date of publication and mention of the grant of the patent: **01.05.2024**
(21) Application number: 17760756.1
(22) Date of filing: 01.03.2017
(51) Int. Cl.: C12Q 1/6869, C12N 15/10

(54) **METHODS AND KITS FOR TRACKING NUCLEIC ACID TARGET ORIGIN FOR NUCLEIC ACID SEQUENCING**
VERFAHREN UND KITS ZUR VERFOLGUNG DES NUKLEINSÄURE-TARGET-URSPRUNGS ZUR NUKLEINSÄURESEQUENZIERUNG
PROCÉDÉS ET KITS DE SUIVI D'UNE ORIGINE CIBLE D'ACIDE NUCLÉIQUE POUR LE SÉQUENÇAGE D'ACIDES NUCLÉIQUES

(30) Priority: 01.03.2016 US 201662301967 P
(43) Date of publication of application: 09.01.2019
(73) Proprietor: Universal Sequencing Technology Corporation, Carlsbad, CA 92008 (US)
(72) Inventor: CHEN, Zhoutao, Carlsbad,CA 92008 (US); PHAM, Long Kim, San Diego,CA 92108 (US); GU, Junchen, San Diego,CA 92122 (US); LEI, Ming, Sharon,MA 02067 (US)
(74) Representative: Barker Brettell LLP
(86) International application number: PCT/US2017/020297
(87) International publication number: WO 2017/151828

(56) References cited:
- WO-A1-2015/189636
- WO-A2-2012/106546
- US-A1- 2014 194 324
- US-A1- 2016 046 985
- US-A1- 2016 369 266
- SASAN AMINI ET AL: "Haplotype-resolved whole-genome sequencing by contiguity-preserving transposition and combinatorial indexing", NATURE GENETICS., vol. 46, no. 12, 19 October 2014 (2014-10-19), pages 1343-1349, XP55279286, NEW YORK, US ISSN: 1061-4036, DOI: 10.1038/ng.3119
- MACOSKO ET AL.: 'Highly Parallel Genome-wide Expression Profiling of Individual Cells Using Nanoliter Droplets' CELL vol. 161, no. 5, 2015, pages 1202 - 1214, XP055323004
- Fang Ge ET AL: "Preferential Amplification of Pathogenic Sequences", Scientific Reports, vol. 5, no. 1, 11 June 2015 (2015-06-11), XP055376868, DOI: 10.1038/srep11047

## Description

### FIELD

The present disclosure relates in general methods for improved nucleic acid sequencing.

### BACKGROUND

The present invention is in the technical field of genomics. More particularly, the present invention is in the technical field of nucleic acid sequencing. Nucleic acid sequencing can provide information for a wide variety of biomedical applications, including diagnostics, prognostics, pharmacogenomics, and forensic biology. Sequencing may involve basic low throughput methods including Maxam-Gilbert sequencing (chemically modified nucleotide) and Sanger sequencing (chain-termination) methods, or high throughput next-generation methods including massively parallel pyrosequencing, sequencing by synthesis, sequencing by ligation, semiconductor sequencing, and others. For most sequencing methods, a sample, such as a nucleic acid target, needs to be processed prior to introduction into a sequencing instrument. For example, a sample may be fragmented, amplified or attached to an identifier. Unique identifiers are often used to identify the origin of a particular sample. Most sequencing methods generate relatively short sequencing reads, ranging from tens of bases to hundreds of bases in length, and cannot generate complete haplotype phase information due to limited sequencing read length. US2014194324 describes a bead based methods for isolating, barcoding and sequencing long individual DNA molecules involving microparticles having transposomes and bead specific barcodes immobilized thereto.

### SUMMARY

The present invention provides methods for tracking nucleic acid target origin by barcode tagging when the targets are broken into smaller pieces. A plurality of nucleic acid sequences which are used as barcodes may be clonally amplified or clonally synthesized on a solid support (e.g., bead, microparticle, slide, plate or flowcell). The design of barcode sequences in this invention allows the creation of billions of different barcodes and each barcode sequence contains features for improving sequencing accuracy. Nucleic acid targets with or without modification are captured *in vitro* by these clonally localized nucleic acid barcode templates on the solid support. Transposase and transposable DNA are used to facilitate the fragmentation and barcode tagging of the nucleic acid targets. Hundreds, thousands or millions of nucleic acid targets can be processed simultaneously in a massively parallel fashion. Each of the targets can be locally captured by a unique group of barcodes in an open bulk reaction without additional partition, such as, with wells, microwells, holes, tubes, spots, nanochannels, droplets, emulsion droplets, capsules, or any other suitable container for comparting fractions of a sample. These captured targets can be broken into smaller fragments, and a target specific barcode sequence will be tagged onto each fragment as an identification of its original target. These nucleic acid target tracking methods can be used for a variety of applications in both whole genome sequencing and targeted sequencing.

The methods presented herein provide several advantages over existing methods, such as Illumina's synthetic long read and 10x Genomics's linked-read. For example, this invention provides millions to billions or more of barcodes which significantly improve the tagging capacity and specificity. The barcode design provides features that reduce sequencing error from long stretch of the same type of nucleotide, i.e., homopolymer sequences and filter out low quality reads so that it improves sequencing quality. Barcodes can be clonally synthesized directly or amplified clonally or semi-clonally using known chemistries (e.g., emulsion PCR method, bridge PCR) on a solid surface. The transposase based fragmentation method simplifies the sample preparation procedure. Unlike all existing methods, the barcode tagging reaction in this invention can be performed in an open bulk solution without additional partition with wells, microwells, holes, tubes, spots, nanochannels, droplets, emulsion droplets, capsules. The procedure is easy to be automated or scaled up for high throughput sample preparation. This invention provides barcode tagging method for not only long nucleic acid samples for applications, such as, haplotype phasing, structure variation detection and copy number study, but also for short nucleic acid samples to track sample uniqueness. The invention is defined by the appended claims. the following disclosure encompasses methods that are not claimed and do thus not form part of the invention.

### BRIEF DESCRIPTION OF THE DRAWINGS

Fig. 1 is a table showing examples of barcode sequence structure and composition.
Fig. 2 illustrates a nucleic acid barcode template.
Fig. 3 shows two different transposable DNA designs, (A) transposon complementary strand with 3' over hang in one piece, (B) transposon complementary strand with a separated complementary linker oligo.
Fig. 4 illustrates the capture of a transposon or transpososome with a complementary 3' overhang on a solid support by hybridization (A) and ligation (B).
Fig. 5 illustrates the capture of a transposon or transpososome with a complementary linker oligo design on a solid support by hybridization (A) and ligation (B).
Fig. 6 is a diagram showing a nucleic acid target (601) which is tagged by transpososomes (602), formed a contiguous transpososome-nuclei acid complex (603), captured by clonal barcode templates (605) on a solid support (604), and then fragmented into barcode tagged fragments (606).
Fig. 7 is a diagram showing multiple nucleic acid targets (701) being tagged by transpososomes (702) simultaneously, captured separately by clonal barcode templates (705) at separated spots on a continuous solid surface (704, e.g. a flow cell surface), or on separate solid supports (e.g. beads or microparticles, not shown), and fragmented into barcode tagged fragments in an open bulk reaction without additional partitions.
Fig. 8 is a diagram showing the encapsulation of a transpososomes-nucleic acid complex (801) and a clonal barcoded bead (802) by a water-in-oil emulsion droplet (805) to generate barcode (803) tagged nucleic acid fragments.
Fig. 9 illustrates two different ways to generate immobilized barcode tagged fragments in an emulsion droplet.
Fig. 10 is a diagram showing the merge of a transpososomes-nucleic acid complex (1001) in a droplet (1003) with a clonal barcode (1004) pool in another droplet (1005) into a combined droplet (1006) to generate barcode tagged fragments (1007).
Fig. 11 illustrates two different ways to generate barcode tagged fragments in a droplet.
Fig. 12 illustrates barcode templates with a transposase binding region (TBR) immobilized on a solid support in different formats. (A) a barcode template with a TBR at one end; (B) immobilized barcode template with a TBR at the free end in double stranded format on a solid support; (C) immobilized barcode template with a TBR at the free end in single stranded format on a solid support, the complementary strand of TBR may be introduced by primer annealing, hybridization and/or primer extension.
Fig. 13 is a diagram showing the binding of transposases (1303) to barcode templates (1301) with a TBR end on a solid support (1302) prior to the capturing and fragmenting a nucleic acid target (1305) for barcode tagging.
Fig. 14 is a diagram showing the binding of transposases (1403) on clonal barcode templates (1401) with a TBR end at different isolated locations on a solid support (1402), each location capturing a different nucleic acid target in parallel, and fragmenting it with barcode tag on the solid support in an open bulk reaction without additional partitions.
Fig. 15 provides an illustration of releasing a copy or copies of immobilized barcode tagged fragments (C) by primer extension (A) and/or PCR amplification (B).
Fig. 16 is an example of Illumina's sequencing library generated from barcode tagged fragments and its sequencing method.
Fig. 17 is an example of Ion Torrent's sequencing library generated from barcode tagged fragments and its sequencing method.
Fig. 18 is an illustration of using released barcode tagged fragments for targeted amplification.
Fig. 19 is an illustration of enriching the gene of interest from a barcode tagged fragment on a solid support directly.
Fig. 20 shows specific examples of barcode and barcode template sequence and structure.
Fig. 21 shows nucleotide content at each position of a specifically designed barcode detected by sequencing.
Fig. 22 lists three examples of MuA transposable DNA designs with attachable end for capture by barcode templates.
Fig. 23 are agarose gel electrophoresis pictures of fragmented genomic DNA after MuA tagmentation reaction. L, 1Kb plus DNA ladder (unit: bp), S, fragmented DNA sample.
Fig. 24 is an electropherogram of a barcode tagged Illumina sequencing library ran on a high sensitivity D5000 screentape on a TapeStation.
Fig. 25 is a diagram showing Illumina sequencing library structure constructed with a barcode tagging method described in this invention.
Fig. 26 shows a Read 1 sequencing read count histogram of same barcode Read 1 read distance to the next alignment.

Transposases in all the figures are illustrated as a tetramer in the transpososome based on the MuA transposition system.

### DETAILED DESCRIPTION

Most commercially available sequencing technologies have limited sequencing read length. Second generation sequencing technologies particularly can sequence only several hundred bases and rarely reach a thousand bases. However, nucleic acid sequences of a gene can span from several kilobases to tens and hundreds of kilobases, which means sequencing read length of tens of kilobases is necessary to successfully determine the haplotypes of all genes. This disclosure provides methods for processing nucleic acid targets into smaller pieces while keeping their origin information with target-specific barcode tags. The processed DNA samples can be used to generate libraries for sequencing applications. The sequencing data can be assembled into full or tandem long reads for haplotype phasing. The methods presented herein provide several advantages over existing methods, such as, Illumina's synthetic long read and 10x Genomics's linked read. For example, this disclosure provides millions and billions or more of barcodes that improves sequencing accuracy by improving the tagging capacity and specificity. Also, unlike existing methods, the barcode tagging reaction in this disclosure may be performed in an open bulk solution without further partitions with wells, microwells, holes, tubes, spots, nanochannels, droplets, emulsion droplets, capsules, etc. The procedure is easy to be automated or scaled up for high throughput sample preparation.

### Barcode design for maximum capacity, sequencing accuracy and utilities

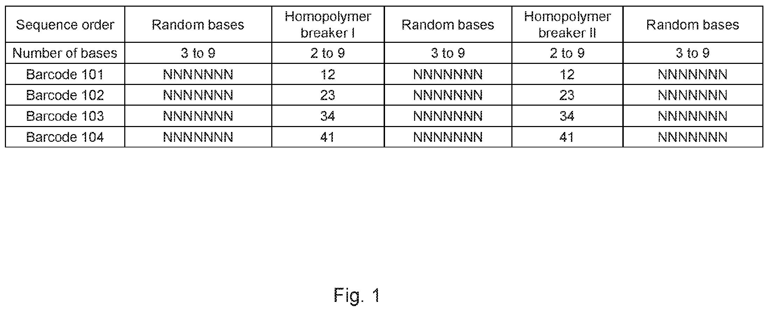

Barcoding methods have been widely used in high throughput sequencing application for sample identification. Barcode designs with completely random or degenerate nucleotide sequences are used for molecular tagging of individual nucleic acid and PCR amplicons. By "barcode" it is meant in general a label that can be associated with (e.g., attached to) a target and convey information (e.g., identity) of that target. By "random" or "degenerate" it is meant a nuclei acid sequence in which one or more positions contain a number of possible bases (e.g., any 2 or 3 or 4 out of A, T, G, C, U). Generally, a barcode can be any nucleic acid sequence of length between 4 to 100 bases, preferably 6 to 25 bases, most common is 6 to 8 bases. The methods and kit disclosed in this disclosure include an improved barcode design to be able to not only offer maximum barcoding capacity, but also improved sequencing accuracy and provide identification for both molecules and samples (e.g., different samples from different patients) at the same time. This disclosure provides a barcode design which contains two or more random nucleotide segments interspersed with predetermined non-homopolymer nucleotide segments (called homopolymer breakers). Each random sequence segment may contain 3 to 9 degenerate bases, preferably 3 to 7 degenerate bases. The length of each random segment may be the same or different. Each homopolymer breaker may have 2 to 9 known bases in length. In one embodiment, the barcode has two random degenerate nucleotide sequencing with one known homopolymer breaker in between. In another embodiment (Fig. 1), the barcode has three random degenerate nucleotide sequences interspersed with two known homopolymer breakers. In some cases, one breaker may have different length from another. In other cases, it may have the same length. In some cases, breaker I has the same sequence as breaker II. In other cases, each breaker has different sequence. In one embodiment, by design, breaker sequences have limited diversity. When one or several bases in a breaker are known, the identity of the rest of the sequence will be known. This barcode design controls the maximum length of homopolymer bases in a barcode. It also has an error check feature to improve the specificity of the barcode identity. Long stretch of homopolymer bases (e.g., 8-mer or above) is very challenging for accurate sequencing. Sequencing errors for very long homopolymer bases are almost inevitable with current available sequencing technologies. By reducing the maximum length of homopolymer bases in a barcode (with homopolymer breakers), sequencing accuracy will improve. Addition of known non-homopolymer nucleotide sequences in a barcode, which can serve as a quality filter, will further improve the sequencing accuracy for the barcode identification. The known sequences in the design can also be used as a high-level identification, such as, sample identification if each sample was prepared with barcodes containing different known sequences. The length of barcode sequences of such a design is minimum 8 bases and can be as many as 100 bases or more, but the preferred length ranges between about 10 to about 50 bases, and ideally about 12 to about 25 bases.

For certain sequencing technologies, such as, Illumina's sequencing by synthesis (SBS) technology, if base sequences for all the molecules are the same at one particular sequencing flow step, it will interfere the signal processing pipeline and tends to lead to higher error rate. In some cases, to avoid all barcode sequences with the same breaker segment, more than one barcode sequence design can be used together. They may have the same barcode structure but with different breaker sequence so that at a particular sequencing flow step, there will be at least two different nucleotide bases presented. In one embodiment in Fig.1, Barcode 101 has three 7-mer random degenerate segments with two 2-mer homopolymer breakers. The base 1 in the breaker can be any one of the 5 nucleotide bases, A, C, G, T, U; the base 2 in the breaker can be any one of the 5 nucleotide bases, A, C, G, T, U, but must be different from base 1 at the same time. To increase the nucleotide diversity at the base 1 position of homopolymer breaker 1 during sequencing with Illumina's SBS technology, one, two or three of Barcode 102, 103 and 104 may be mixed with Barcode 101 together as the final barcode design. A barcode design with mixture of Barcode 101, 102, 103 and 104 may generate maximum 4×10²² permutations (T and U are unable to differentiated by current sequencing technology, so all together four nucleotide bases are considered in the permutation, and since homopolymer breakers are fixed sequences, only four possibilities are considered.), i.e., 1.76×10¹³ different barcode sequences.

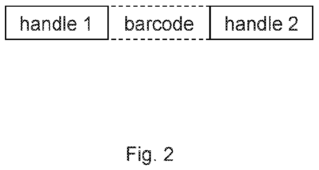

A "barcode template", which contains a barcode sequence, flanked by at least one handle sequence at one end or two handle sequences at both ends (Fig. 2). The handle sequences can be used as binding sites for hybridization or annealing, as priming sites during amplification, or as binding site for sequencing primers or transposase enzyme. Barcode templates including properly designed barcode and two handles may be generated from oligonucleotides obtained from standard synthesis or from an oligonucleotide array.

The barcode templates (Fig. 2) can be clonally immobilized by clonal amplification or synthesis to a suitable solid support, such as a bead, a microparticle, a slide, a plate, or a flowcell, wherein said solid support has a plurality of the same barcode template with one unique barcode sequence on the surface ("clonal barcode templates"). The clonal amplification methods may include bridge polymerase chain reaction (PCR), emulsion PCR amplification, isothermal amplification with template walking and recombinase polymerase amplification (RPA) reaction, etc. For optimal clonal amplification on the solid support with barcode templates, limited dilution may be used. For emulsion based amplification, dilute barcode templates so that majority of the emulsion droplets contain only one barcode template. More than one barcode template for a cluster or a droplet in a clonal amplification reaction is not optimal, but they can be well tolerated in some applications if the number of different barcode templates in one clonal reaction is not high. This is referred as semi-clonally amplification which results in "semi clonal barcode templates". For emulsion PCR amplification, based on the Poisson distribution, if mix the beads and barcode templates in a 1:1 ratio, it will proximately end up with 36.8% beads with one barcode template theoretically, 18.4% beads with two barcode templates, 8% with three or more barcode templates. The percentage of good beads (with one or two barcode templates) is 87.3% among all positive beads, but there will be about 36.8% of beads without barcode templates which will be wasted. However, if the bead/barcode template ratio increases to 2:1, there will be about 30.3% one barcode, 7.6% two barcodes, but only 1.4% with three or more barcodes. The good bead ratio will increase to 96.3% among positive beads but with more than half beads wasted. When beads or microparticles are used as solid support, these beads or microparticles may be of uniform size or heterogeneous size. For better results, the diameter of bead or microparticle should be controlled between 200nm to 50µm, preferably 1µm to 15µm, though it can be as small as 40nm or as large as 100µm. In addition, the beads or microparticles may be magnetic for ease of handling. The beads or microparticles may also be porous or non-porous. In some embodiment, additional procedure may be used to enrich only beads or microparticles with amplified barcode templates. In other embodiment, enrichment procedure may not be required. Beads or microparticles without barcode templates can serve as spacer in the downstream reactions._For bridge PCR, dilute barcode templates so that majority of cluster are formed from one barcode template. For the barcode clusters on a solid surface, such as a flow cell surface, the cluster size should be controlled between 50nm to 200µm (diameter), preferably 100nm to 100µm, and the cluster separation distance should be at least larger than the cluster size, ideally larger than the length of the longest nucleic acid target, to avoid one nucleic acid target being captured by two or more barcode clusters. The general rule is that the longer the nucleic acid target is, the larger the bead size or barcoded cluster size as well as the bead or cluster separation distance. The distance between clusters can be controlled by limited dilution of barcode templates or by specially designed array-like surface. Clonally amplified barcode templates can be double stranded or be denatured to be only single stranded on any type of solid support.

In some cases, a single stranded barcode template polynucleotide can be directly clonally synthesized on a solid support, such as, with reverse synthesis and split- and-pool method (Macosko et al., 2015) without clonal amplification.

### Capture nucleic acid-transpososome complexes with clonally barcoded solid support without additional partition for barcode tagging of the nucleic acid

The present disclosure provides methods and kits that capture nucleic acid targets, which are bound by transpososomes, to a clonally barcoded solid support. The captured nucleic acid target may then be fragmented and tagged with barcode sequences on the barcoded solid support.

The term "transposase" as used herein refers to an enzyme that is a component of a functional nucleic acid protein complex capable of transposition and which is mediating transposition. The term "transposase" also refers to integrases from retrotransposons or of retroviral origin. It also refers to both wild type enzymes and mutant enzymes and fusion enzyme with tag, such as, GST tag, 6xHis-tag, etc.

The term "transposon", as used herein, refers to a nucleic acid segment that is recognized by a transposase or an integrase enzyme and is an essential component of a functional nucleic acid-protein complex capable of transposition. It refers to both wild type and mutant transposon.

A "transposon end sequence" as used herein refers to the nucleotide sequences at the distal ends of a transposon. The transposon end sequences are responsible for identifying the transposon for transposition; they are the DNA sequences required to form a transpososome and to perform a transposition reaction.

A "transposable DNA" as used herein refers to a nucleic acid segment that contains at least one transposon unit.

The term "transpososome" as used herein refers to a transposase enzyme non-covalently bound to a double stranded nucleic acid (i.e., transposon).

A "transposition reaction" as used herein refers to a reaction where a transposon inserts into a target nucleic acid. Primary components in a transposition reaction are a transposon, a transposase or an integrase enzyme, and its target nucleic acid.

The term "transpososome-nucleic acid complex" or "nucleic acid-transpososome complex" as used herein refers to strand transfer complex (STC), a stable_nucleic acid-protein complex of transpososome and its target nucleic acid into which transposons insert.

A "transposase binding region" as used herein refers to the nucleotide sequences that are always within the transposon end sequence where a transposase specifically binds when mediating transposition. The transposase binding region may comprise more than one site for binding transposase subunits.

A "transposon joining strand" as used herein means the strand of the double stranded transposon DNA that is joined by the transposase to the target nucleic acid at the insertion site.

A "transposon complementary strand" as used herein means the complementary strand of the transposon joining strand in the double stranded transposon DNA.

The method and materials of the disclosure are exemplified by employing *in vitro* MuA transposition (Haapa et al. 1999 and Savilahti et al. 1995). Other transposition systems can be used, e.g., Ty1 (Devine and Boeke, 1994), Tn7 (Craig, 1996), Tn10 and IS10(Kleckner et al. 1996), Mariner transposase (Lampe et al., 1996), Tc1 (Vos et al., 1996, 10(6), 755-61), Tn5 (Park et al., 1992), P element (Kaufman and Rio, 1992) and Tn3 (Ichikawa and Ohtsubo, 1990), bacterial insertion sequences (Ohtsubo and Sekine, 1996), retroviruses (Varmus and Brown 1989), and retrotransposon of yeast (Boeke, 1989).

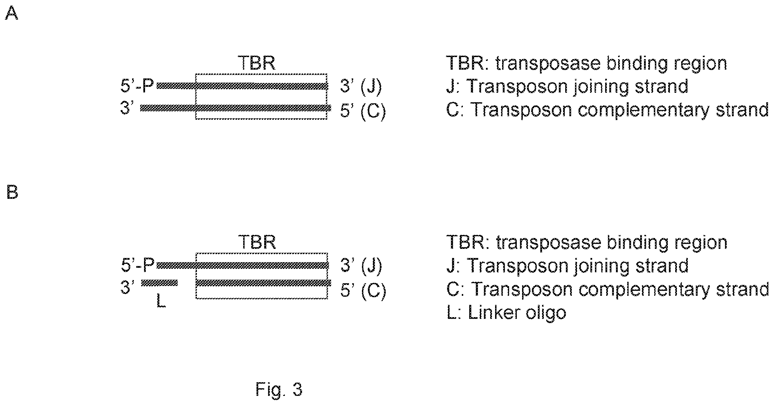

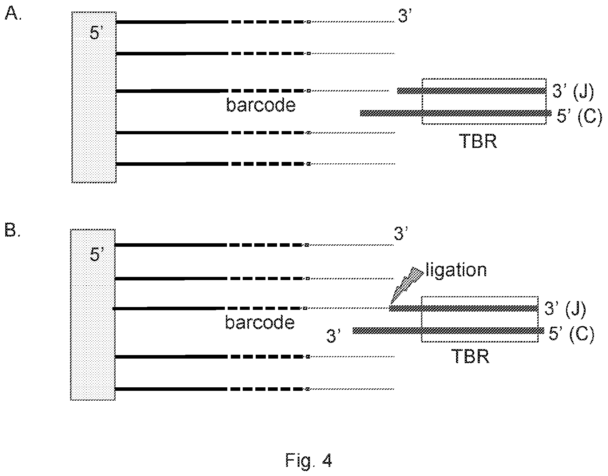

In the present disclosure, a transposable DNA may comprise only one transposon end sequence (Fig. 3). The transposon end sequence in the transposable DNA sequence is thus not linked to another transposon end sequence by a nucleotide sequence, i.e., the transposable DNA contains only one transposase binding region. In addition, the 5' end of joining strand of the transposable DNA has a phosphate, which can ligate to a 3' end of any DNA strand with -OH group. The 3' end of the transposon complementary strand may overhang out as a single stranded DNA partially (Fig. 3A). The protruding ssDNA sequence may be complementary to the 3' end of the immobilized polynucleotide on a solid support (Fig. 4A) so that it can be annealed or hybridized together. The length of the protruding end can be any number of bases from 1 up to the length of the immobilized polynucleotide on the solid support. The 5' end of the transposon joining strand can ligate to the polynucleotide on the solid support with or without the presence of the transposases on the transposable DNA (Fig. 4B).

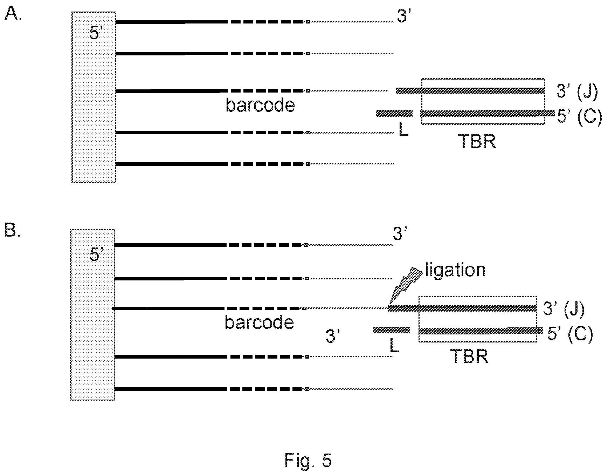

In some cases, the 3' end of the transposon complementary strand may be shorter than the 5' end of joining strand of the transposable DNA (Fig. 3B). A linker oligonucleotides (L) bound to the 5' end of joining strand is needed. It has complementary sequences to the 5' end of joining strand of the transposable DNA and complementary sequences to the 3' end of the immobilized polynucleotide on a solid support (Fig. 5). The 5' end of the transposon joining strand can ligate to the polynucleotide on the solid support with or without the presence of the transposases on the transposable DNA (Fig. 5B). In some cases, the linker oligonucleotides may be bound with transposable DNA (Fig. 3B). In some cases, the linker oligonucleotides may be bound with the immobilized polynucleotides on a solid support. In some cases, the linker oligonucleotides may be added only when ligation reaction happens to join the 5' end of joining strand of the transposable DNA to 3' end of the immobilized polynucleotide on a solid support.

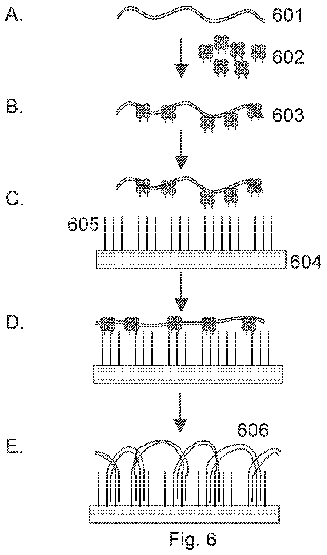

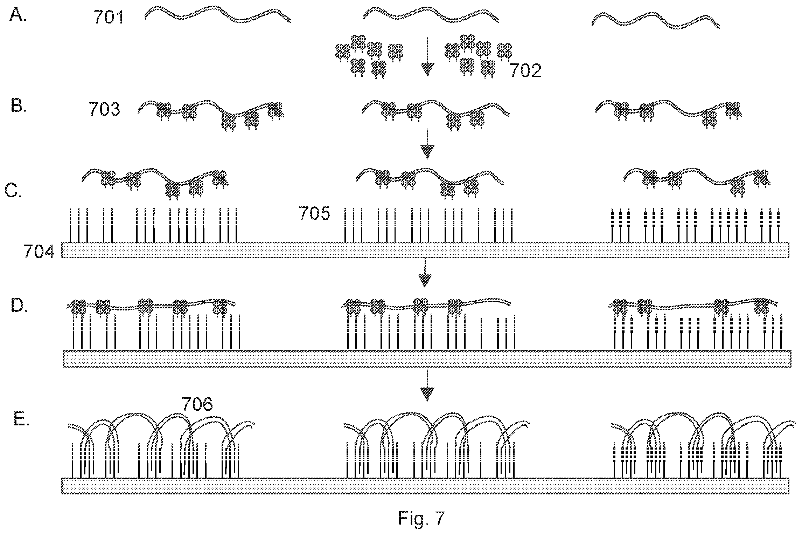

A method for fragmenting and barcoding nucleic acid samples is described as following (Fig. 6 and Fig. 7). A double stranded nucleic acid target (601) reacts with transposable DNA and transposase to form a transpososome-nucleic acid complex (603). Each key component, nucleic acid target, transposable DNA, and transposase, may be added into the reaction at the same reaction step without pre-incubation of any two of the three components first. The length of double stranded nucleic acid target may be in the range from about 100bp to about 1Mb or more. The longer the length of the nucleic acid targets, the better the result for phasing application. The transposable DNA may be designed as the transposable DNA in Fig. 3. The transposable DNA may incubate with transposase in a condition to form the transpososome first (602) before reacting with a nucleic acid target (601).

Both Tn5 transpososome and MuA transpososome have been previously described to simultaneously fragment DNA and introduce adaptors at high frequency *in vitro,* creating sequencing libraries for next-generation DNA sequencing (Adey et al 2010, Caruccio et al 2011, and Kavanagh et al 2013). These specific protocols remove any phasing or contiguity information as a result of the fragmentation of the DNA. However, in these protocols after DNA reaction with transpososomes, a column purification, a heat treatment step, a protease treatment or an incubation with a SDS solution was necessary to release the transposase from the transpososome-DNA complex so that DNA becomes fragments. However, the DNA string bound with transpososomes, known as strand transfer complexes, are very stable under natural condition (Surette et al 1987, Mizuuchi et al 1992, Savilahti et al 1995, Burton and Baker 2003, Au et al 2004, Amini et al 2014), and so is the DNA string with transpososome (603) in Fig. 6.

The DNA strings with transpososomes are incubated with barcoded solid support (604) as described in Fig. 6. The barcode templates (605) on the solid support may be denatured to become single stranded first or start as single stranded. The 3' protruding ends of the transposon complementary strand from the transpososomes on the DNA string can be captured by the single stranded barcode templates on the solid support by hybridization (Fig. 6D). A ligation reaction, e.g., with T4 DNA ligase may be used to ligate the transposon joining strand to 3' end of barcode template on the solid support as Fig. 4B. The transposases on the captured DNA string may then be released with a heat treatment step, such as at about 65°C to about 75°C for approximately 5-10 minutes, or utilizing protease or a protein denaturing agent, e.g. SDS solution, guanidine hydrochloride, urea, etc. A DNA polymerase may be used to fill in the gaps left during the transposition reaction. Each nucleic acid fragment (606) may contain a barcode sequence after the reaction.

Many double stranded nucleic acid targets may react with transposable DNA and transposase simultaneously in various concentrations to generate many complexes. When many nucleic acid-transpososome complexes (Fig. 7B) presented in a reaction, limited dilution may be used to have DNA string captured separately on a barcoded solid support so that one clonal barcode region captures a limited number of DNA string. The solid support (704) may be a continuous surface as in a slide, a plate or a flow cell with isolated clonally or semi-clonally immobilized barcode template clusters or wells or areas (shown as different patterns, 705). It can also be physically separated as individual bead or microparticle. Each bead or microparticle has a plurality of barcode templates with unique sequence. The major advantage in the present disclosure is that capture of DNA string with transpososomes can occur in an open bulk reaction without additional partitions with wells, microwells, spots, nanochannels, droplets, emulsion droplets or capsules, etc. More than one DNA string may be captured by one clonally barcoded bead or microparticle, or clonally barcoded cluster on a slide, plate or flowcell. By controlling reaction concentration, the chance of nucleic acid from the same location of a genome or chromosome being captured by the same barcode will be very limited. Millions and billions or more of barcode templates may be used to prepare barcoded beads or other solid support, which will further minimize the chance of the same barcode tagging to the nucleic acid targets from the same location of a genome or chromosome.

### Encapsulating nucleic acid-transpososome complexes and clonally barcoded beads or microparticles in water-in-oil emulsion droplets

This disclosure provides a method to encapsulate nucleic acid targets bound with transpososomes and clonally barcoded beads or microparticles in water-in-oil emulsion droplets, and further generate barcode tagged nucleic acid fragments.

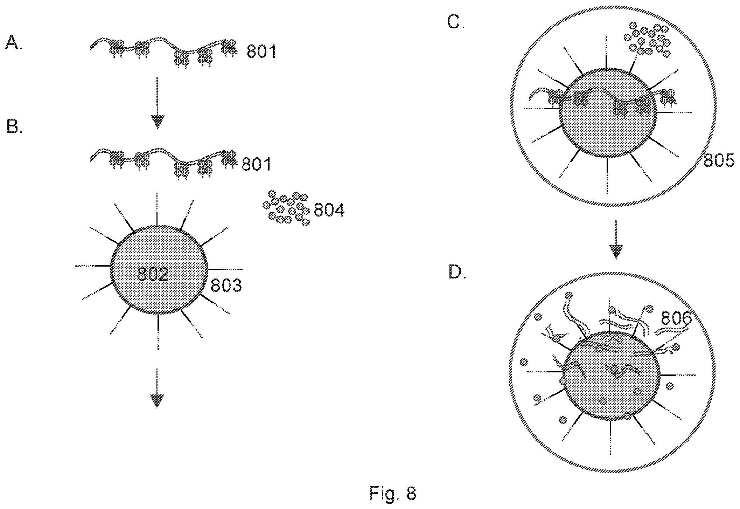

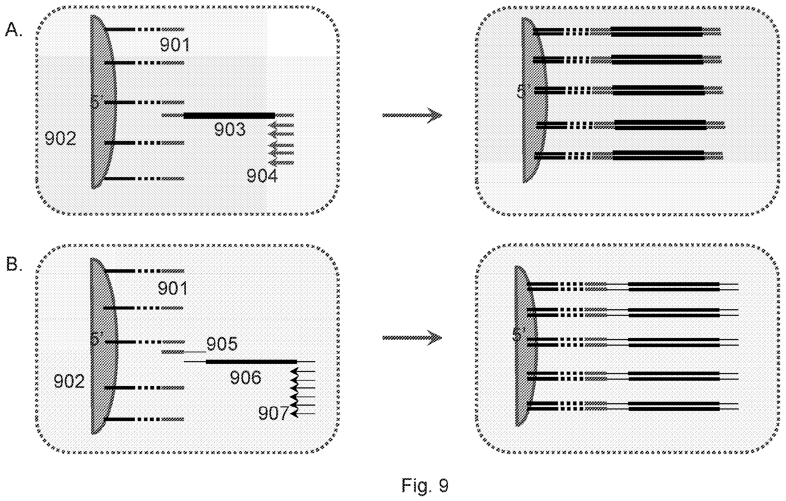

The DNA strings with transpososomes, i.e. the contiguous nucleic acid-transpososome complexes, which are generated as described previously in this disclosure (Fig. 6B), are used as starting material (801) in this method (Fig. 8A). Beads or microparticles (802) with clonal barcode templates (803), are provided by clonal amplification method or direct synthesis method as described in the previous section in this disclosure. Additional enzymes and substrates (804), such as, DNA polymerase, dNTP and primers are provided in an aqueous solution. Water-in-oil emulsion droplets (805) are generated in such condition that one to a few nucleic acid targets are mixed with one barcoded bead or microparticle in one droplet. Limiting titration and/or partitions of nucleic acid string and barcode beads or microparticle can be used here based on the Poisson distribution. The fewer the number of nucleic acid targets per droplet, the higher power for phasing application. After a heat treatment, such as at about 65°C to about 75°C for about 5 -10 minutes, transposase will release from the transpososome-nucleic acid complex and nucleic acid target breaks into smaller fragments (806). When still in a water-in-oil droplet, a DNA polymerase may be used to fill in the gaps left during the transposition reaction. Furthermore, a primer extension reaction or PCR amplification reaction can drive the nucleic acid fragments onto barcoded bead or microparticle to generate barcode tagged nucleic acid fragments. In one embodiment, there are overlap nucleotide sequences between 3' end of single stranded barcode template (901) and one end of nucleic acid fragments (903) in Fig. 9A. A direct primer extension or PCR amplification reaction is able to add the nucleic acid fragments onto the solid support (902). In another embodiment, there are no overlap nucleotide sequences between single stranded barcode template (901) and the ends of nucleic acid fragments (906) in Fig. 9B. A bridging oligonucleotide (905), which has one end overlap with 3' end of barcode template and another end overlap with one end of nucleic acid fragments, is used to drive the nucleic acid fragments onto the barcoded solid support (902) via primer extension or PCR amplification. The beads or microparticles with immobilized barcode tagged nucleic acid fragments can then be released from emulsion droplets for further downstream processing.

It should be noted that partitions can be used in connection with these or other embodiments. The term "partition," as used herein, may be a verb or a noun. When used as a verb (e.g., "to partition," or "partitioning"), the term generally refers to the fractionation (e.g., subdivision) of a species or sample (e.g., a polynucleotide) between vessels that can be used to sequester one fraction (or subdivision) from another. Such vessels are referred to using the noun "partition." Partitioning may be performed, for example, using microfluidics, dilution, dispensing, vortexing, filtering and the like. A partition may be, for example, a well, a microwell, a hole, a droplet (e.g., a droplet in an emulsion), a continuous phase of an emulsion, a test tube, a spot, a capsule, a bead, a surface of a bead in dilute solution, or any other suitable container for sequestering one fraction of a sample from another. A partition may also comprise another partition.

### Encapsulating nucleic acid-transpososome complexes with clonal barcode oligonucleotide pools in water-in-oil emulsion droplets

This disclosure provides a method to encapsulate nucleic acid targets bound with transpososomes and clonal barcode oligonucleotide pools in water-in-oil emulsion droplets, and further generate barcode tagged nucleic acid fragments.

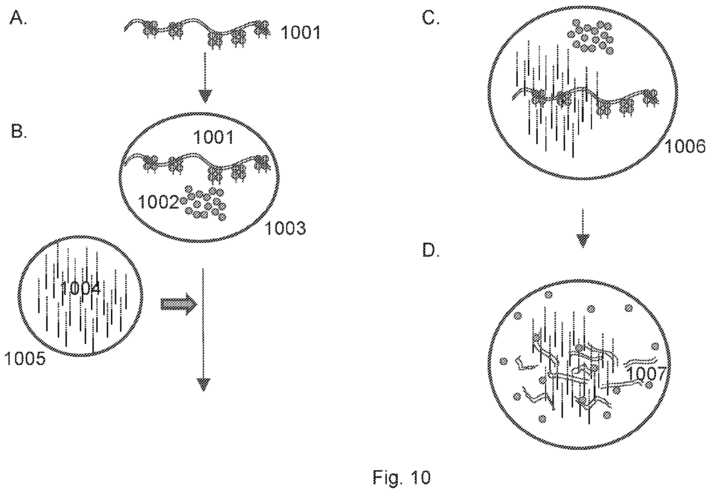

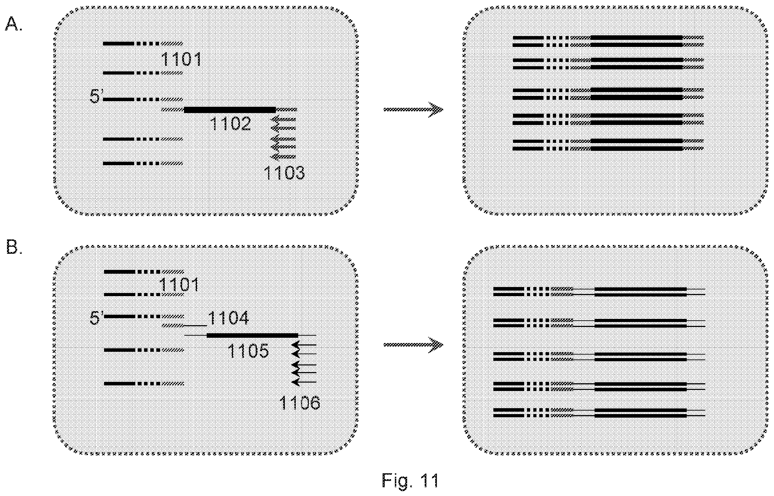

The DNA strings with transpososomes, i.e. the contiguous nucleic acid-transpososome complexes, which are generated as described previously in this disclosure (Fig. 6B), are used as starting material (1001) in this method (Fig. 10A). Additional enzymes and substrates (1002), such as, DNA polymerase, dNTP and primers are provided in an aqueous solution. Water-in-oil emulsion target droplets (1003) are generated in such as condition that one to a few DNA strings are present in most droplets (e.g., by limiting titration or partitions based on Poisson distribution). The fewer the number of DNA strings per droplet, the higher power for phasing application. Clonal barcode templates (1004) may be provided as clonal barcode oligonucleotide pools in water-in-oil droplets (1005). In an embodiment, merge one target droplet with one barcode droplet using a T-shape or Y-shape valve or other means to generate a new combined water-in-oil emulsion droplet (1006) containing one DNA string and one barcode pool. After a heat treatment, such as at about 65°C to about 75°C for about 5 - 10 minutes, transposase will release from the transpososome-nucleic acid complex and the nucleic acid target breaks into smaller fragments (1007). When still in a water-in-oil droplet, a DNA polymerase may be used to fill in the gaps left during the transposition reaction. Furthermore, a primer extension reaction or PCR amplification reaction can attach the barcode sequence onto the nucleic acid fragments to generate barcode tagged nucleic acid fragments. In one embodiment, there are overlap nucleotide sequences between 3' end of single stranded barcode template (1101) and one end of nucleic acid fragments (1102) in Fig. 11A. A direct primer extension or PCR amplification reaction is able to attach the barcode sequence to the nucleic acid fragments. In another embodiment, there are no overlap nucleotide sequences between single stranded barcode template (1101) and the ends of nucleic acid fragments (1105) in Fig. 11B. A bridging oligonucleotide (1104), which has one end overlap with 3' end of barcode template and another end overlap with one end of nucleic acid fragments, is used to link the barcode template to the nucleic acid fragments via primer extension or PCR amplification. The barcode tagged nucleic acid fragments can then be released from emulsion droplets for downstream applications.

### Capture nucleic acid with immobilized clonally barcoded transpososomes for barcode tagging of nucleic acid without additional partition

This disclosure provides methods to capture nucleic acid targets with immobilized clonally barcoded transpososome complexes, fragment the captured nucleic acid and attach the barcode sequence to the fragments without additional partition.

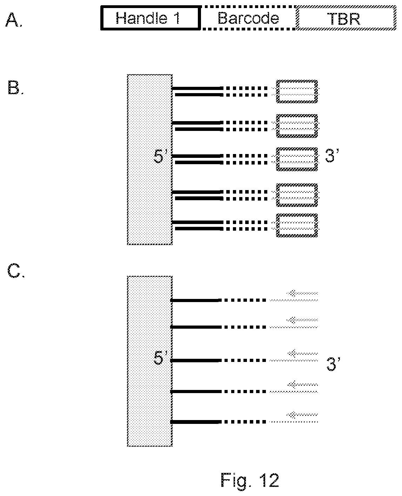

The barcode template used for this application contains both barcode sequence and a transposase binding region. In one embodiment, the barcode template may have the structure as the Fig. 12A. A barcode sequence is flanked by two handles, handle 1 and handle 2. The handle 2 contains a transposase binding region. A solid support with clonal barcode templates (Fig. 12B) may be prepared as described previously in this disclosure with either clonal amplification method or direct synthesis method. Double stranded barcode templates on a solid support can be generated with clonal amplification methods. When necessary, single stranded barcode templates on a solid support may be converted to partially or fully double stranded barcode templates with a primer annealing or primer extension reaction using partial or full sequence of handle 2 as primer (Fig. 12C).

A method for clonal barcode tagging and fragmentation of nucleic acid sample is described as following but not claimed . A solid support (1302) with double stranded barcode template including transposase binding region (1301) may incubate with transposase (1303) and nucleic acid target (1305) simultaneously or separately. In one embodiment, transposase (1303) may incubate with a barcode solid support (1302), bind to TBR of the barcode templates and form transpososome on the solid support (1304). Nucleic acid target may be captured by the immobilized transpososome. After a heat treatment step, such as at about 65°C to about 75°C for approximately 5 -10 minutes, a protease treatment or incubation with a protein denaturing agent, e.g. SDS solution, guanidine hydrochloride, urea, etc., transposase will be released from the solid support and fragmented nucleic acid target is exposed (1307). Additional reaction with a DNA polymerase may perform to fill in the gaps generated during transposition reaction.

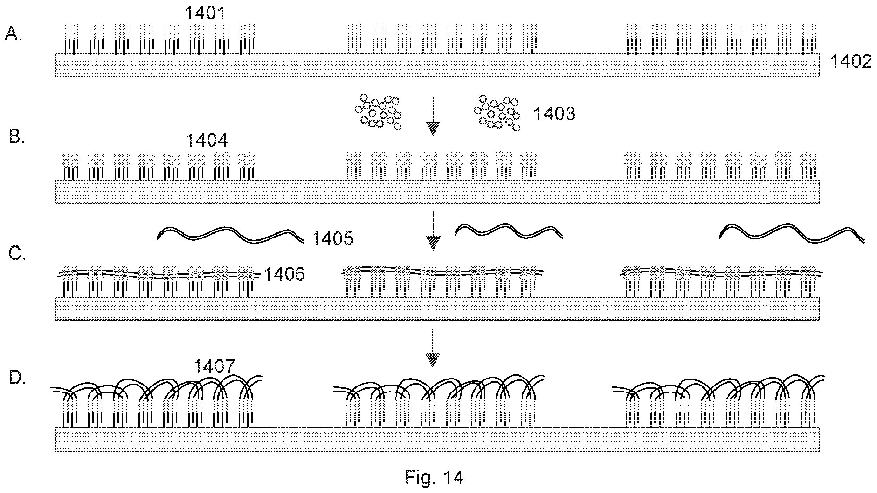

In an open bulk reaction when many different nucleic acid targets present, a solid support or solid supports with many different clonal barcode templates prepared according to previously described procedure in this disclosure will be used to clonally capture each nucleic acid target. Limited dilution of the nucleic acid targets may be used. However, no additional partitions with wells, microwells, spots, nanochannels, droplets, emulsion droplets or capsules is necessary. The solid support (1402) may be separated as individual bead or microparticle (Fig. 14). Each bead or microparticle has a plurality of barcode templates with unique sequence (shown as different pattern for 1401). Also described but not claimed, The solid support may also be continuous as with a slide, plate or flowcell. Different barcoded templates are clonally amplified at different location of the same slide, plate or flowcell surface. More than one nucleic acid target may be captured by one barcode templated beads or microparticle, or barcode templated region on the slide, plate or flowcell. By controlling reaction concentration, the chance of nucleic acid targets from the same location of a genome or chromosome captured by the same barcode will be very limited. Millions and billions barcode templates may be used to prepare barcoded solid support, which will further minimize the chance of the same barcode tagging to the nucleic acid targets from the same location of a genome or a chromosome.

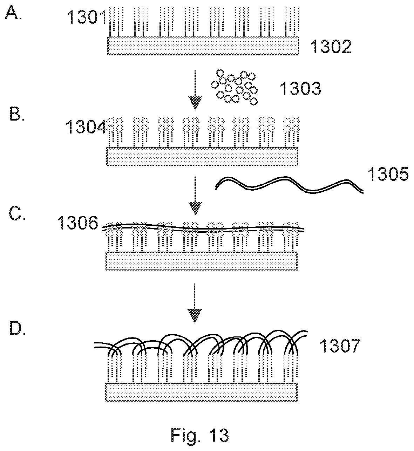

Disclosed but not claimed, Transposases can pre-loaded on the barcoded solid support in the method depicted In Fig. 13 and Fig. 14. However, barcode density on the solid support may have significant effect on the transposition reaction efficiency and fragment size of barcode tagged nucleic acid targets. More transposase may be wasted for the same amount of products generated compared with the method depicted in Fig. 6 and Fig. 7.

### Capture nucleic acid with immobilized clonally barcodes for barcode tagging of nucleic acid without additional partition

The method in the previous section uses transposition reaction for capturing nucleic acid targets to a clonally barcoded solid support without additional partition. Alternatively, nucleic acid targets can be captured to a clonally barcoded solid support via primer extension reaction with or without strand displacement. The distal end of immobilized barcode template may contain a string of degenerate nucleotides ranging from 6 bases to 20 bases, which can be used as a random primer and annealed to nucleic acid target for target capture. Further primer extension reaction using a DNA polymerase with or without strand displacement function will create a copy or copies of portions of targeted nucleic acid with barcode attached.

### Releasing clonally barcode tagged nucleic acid fragments to generate sequencing library

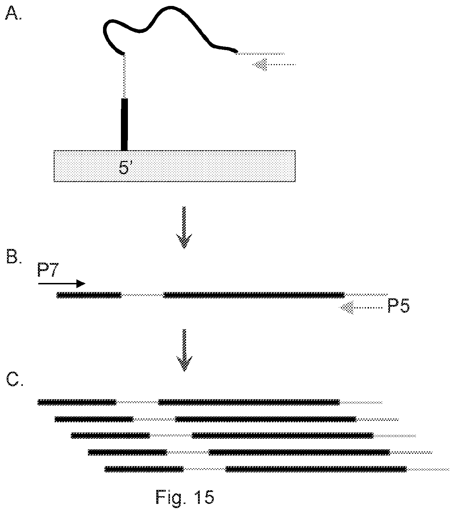

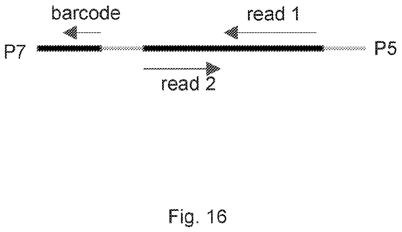

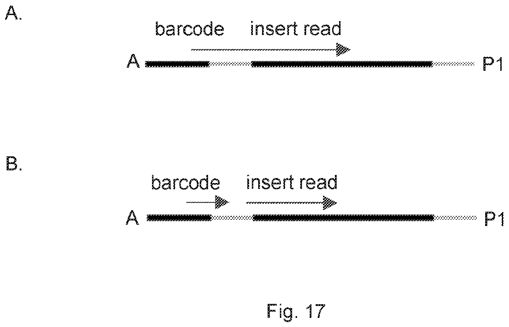

The barcode tagged fragments (706, 1407) are immobilized on the solid support. They may be released from the solid support in many ways. In one embodiment, a cleavable link or a rare restriction site may be included in the oligonucleotide sequence which is attached to the solid support. With a cleavage reaction or a restriction enzyme digestion, the barcode tagged fragments can be released from the solid support. In some cases, a primer extension may be performed to make a copy or copies of the barcode tagged fragments (Fig. 15). Further PCR amplification with primers which are specific for any sequencing platform, e.g., P5 and P7 primers for Illumina's SBS library, or P1 and A primers for Ion Torrent's library, may generate sequencing ready libraries for the specific sequencing platform. When a library is being made by releasing the barcode tagged fragments from the solid support, a primer with sample specific index may be used. In some cases, the known sequences in the barcode template may be used as sample specific index. The released barcode tagged fragments with sample specific index can mix with tagged fragments from other samples with their own sample specific index together for further downstream workflow in order to increase sample preparation throughput and simplify the process. The constructed libraries can be sequenced to generate sequences of both barcode and nucleic acid target. In one embodiment, libraries for Illumina's SBS sequencing chemistry are generated from the barcode tagged nucleic acid fragments (Fig. 16). Barcode sequence may be detected as a long custom index read on Illumina's sequencing platforms. Nucleic acid target sequences may be generated as single end reads or paired end reads as needed. In another embodiment, libraries for Ion Torrent's sequencing chemistry are generated from the barcode tagged nucleic acid fragments (Fig. 17). In one embodiment, barcode sequence and nucleic acid target sequence may be generated from a single long read (Fig. 17A). In another embodiment, barcode sequences may be generated first with sequencing primer A. The chip may be removed from the instrument. The double strand DNA on the chip may be denatured with a low concentration sodium hydroxide solution and leave only the single stranded DNA attached to the Ion Sphere Particles. A second sequencing primer may be used with sequencing polymerase on the same chip to generate sequencing read of the nucleic acid target (Fig. 17B).

### Assemble barcode sequencing reads into long reads

This disclosure provides methods and kit to clonally barcode tag nucleic acid samples in an open bulk reaction without sophisticated compartmentation or partition scheme as other methods. The barcode tagged fragments may be from a whole genome sample. The sequencing reads generated from these barcode tagged fragments may be used to assemble the whole genome as a haploid sequencing method.

The sequencing reads generated from these barcode tagged fragments contain the barcode information which can be used to identify the target origin of these fragments. These short sequencing reads with the same barcode can be grouped together and comprise many short tandem reads spread along the original nucleic acid targets. They provide useful long range linkage information to be used for haplotype phasing. The longer the original nucleic acid targets are, the longer the tandem reads will be, the more useful they are for phasing application. An analysis pipeline which can be developed for full genome assembly or structural variation analysis using these barcode reads for both de novo sequencing and resequencing. In one case, all the sequencing reads may be used for standard shotgun assembly analysis to establish many initial contigs first. The barcode information can then be used to phase the initial contigs into much longer contigs. One of the embodiments in this disclosure is to generate barcode solid support with clonal amplification. Even with limited dilution method, more than one barcode template may be clonally amplified on the same beads or microparticles or at close locations on the slide or flowcell. It is also possible that one barcode templates may be clonally amplified on more than one solid support or solid support surface area to create replicated barcode solid supports. However, the barcode templates designed in this disclosure can generate millions and billions or more of different barcodes, the level of polyclonal barcode solid support and duplicated barcode solid support generated in the process will not significantly interfere with the assembly of the barcode tagged reads overall.

### Targeted sequencing with barcode tagged fragments

This disclosure also provides methods to use these barcode tagged fragments for targeted sequencing application according to the following.

In one case, the region of interest, such as HLA genes or CYP2D6 gene, may be amplified as long range PCR products. These long range PCR products can be used as DNA targets directly with the barcode tagging methods described in this disclosure. The tandem long reads generated from the described method can phase back these long range PCR fragments accordingly.

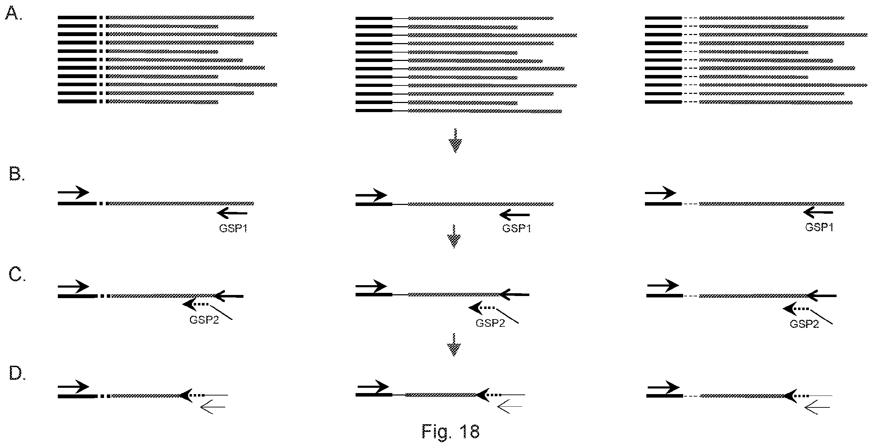

In some cases, a whole genomic DNA sample may be barcode tagged using the methods described in this disclosure first. In one embodiment, these barcode tagged genomic DNA fragments may be released from the solid support as priming extension products or cleaved from the solid support biochemically (Fig. 18A). A first set of gene specific primers (GSP1) for the genes of interest may be used for a round of primer extension or a few rounds of amplification with a common primer on the barcode tagged fragments. The number of GSP1 primers may be between about 3 and about 40,000. A second set of gene specific primers (GSP2) which are nested inside the GSP1 priming products may be used with the common primer on the barcode tagged fragments to further amplify the genes of interest. The number of GSP2 primers may be between about 3 and about 40,000 as the number of the GSP1 primers. The use of GSP2 primers can improve the on target rate significantly. When processing multiple samples are necessary, a sample specific index may be included as a tail of the GSP2 primers or the common primer on the barcode tagged fragments, so that amplification products from different samples can be mixed together later for further downstream procedure, such as sequencing.

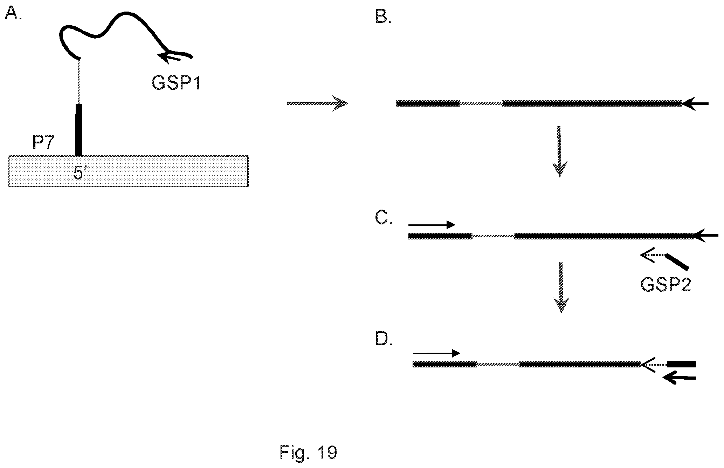

In another embodiment, these barcode tagged genomics DNA fragments stay on the solid support (Fig. 19A). A first set of gene specific primers (GSP1) for the genes of interest may be used for a round of primer extension directly with the fragments on the solid support. The number of GSP1 primers may be between about 3 and about 40,000. The primer extended copies of the targeted genes may be denatured and released from the solid support. Additional amplification with GSP2 primers and a common primer on the barcode tagged fragments can perform to enrich the fragments with the genes of interest. The number of GSP2 primers may be between about 3 and about 40,000 as the number of the GSP1 primers. When processing multiple samples are necessary, a sample specific index may be included as a tail of the GSP2 primers or the common primer on the barcode tagged fragments, so that amplification products from different samples can be mixed together later for further downstream procedure, such as sequencing.

These barcode tagging methods may be used for phasing the targeted gene, genes, or exome. These barcode tagging methods may also be used as a tool for differentiating the duplicated reads in the targeted sequencing application. This method improves sequencing assay detection limit on heterogeneous samples, e.g., somatic mutation detection in a cancer biopsy sample or circulating tumor cell/DNA.

An embodiment of the present disclosure is a barcode template that comprises a barcode sequence and two handle sequences flanking the barcode sequence. The barcode sequence comprises one or more segments of random nucleotide sequence with one or more segments of known nucleotide sequence. In some embodiments, each handle sequence is approximately between about 10 nucleotides and about 100 nucleotides in length. In other embodiments, the handle sequences comprise sequences for priming and/or hybridization. Further, the handle sequences may comprise transposon end sequences. In some instances, the barcode sequence is between about 6 nucleotides and about 100 nucleotides in length. The known sequence in the barcode sequence is between about 2 nucleotides and about 50 nucleotides in length. The known sequence in the barcode sequence may be used as quality filter to remove error prone sequencing reads.

Another embodiment of the present disclosure is a method of clonally barcode tagging nucleic acid targets comprising: providing a solid support having clonal barcode templates immobilized thereon; providing a transposable DNA, wherein said transposable DNA has its 5' end of transposon joining strand ligatable to the 3' end of said immobilized barcode template; applying nucleic acid targets to said transposable DNA and transposase to form DNA-transpososome strings in solution; hybridizing the DNA-transpososome strings with said solid support having barcode templates, wherein said 5' ends of joining strand of transposable DNA ligate to barcode templates, without any additional compartmentalization; and applying a heat treatment, a protease or a protein denaturing agent, e.g. SDS solution, guanidine hydrochloride, urea, etc., to release said transposase from said transpososomes. In some embodiments, the transposable DNA has one transposon end sequence from wildtype or mutant Tn5 or MuA transposon DNA; wherein said transposase is one of wildtype or mutant Tn5 or MuA transposase. The 5' end of transposon joining strand of said transposable DNA has phosphate suitable for ligation. The 3' end of transposon complementary strand of said transposable DNA has a protruding end and the protruding end comprises complementary nucleotide sequences of the said barcode template on the solid support; and the said transposable DNA can hybridize to the said barcode template; and the 3' end of barcode template is ligatable with 5' end of transposon joining strand directly or after modification with an enzyme. The length of said protruding end is about 1 bases, about 3 bases, about 5 bases, about 10 bases, about 15 bases, about 20 bases, about 25 bases, about 30 bases or as long as the length of the immobilized oligonucleotide on the solid support. The number of said nucleic acid molecules are at least about 10², 10³, 10⁴, 10⁵, 10⁶ wherein said DNA-transpososome strings are diluted in the reaction solution before hybridize to the said solid support. The hybridization reaction may be performed with further compartmentalization in plates, microwells or nanochannels.

Another embodiment of the present disclosure is a method of clonally barcode tagging nucleic acid targets comprising providing a solid support having clonal barcode templates immobilized thereon; providing the distal end from the solid support of said barcode templates has a transposon binding region; providing the said barcode templates on the solid support is double stranded for the transposable DNA end; applying transposase and nucleic acid targets to said solid support with immobilized barcode templates to form DNA-transpososome string on the surface of solid support without any additional compartmentalization; and applying a heat treatment, a protease or a protein denaturing agent to release said transposase from the transpososomes. The transposon binding region is from wildtype or mutant Tn5 or MuA transposon DNA; wherein said transposase is one of wildtype or mutant Tn5 or MuA transposase. The number of the nucleic acid targets is in the range of about at least 10², 10³, 10⁴, 10⁵, or 10⁶. The nucleic acid targets are diluted in the reaction solution before reaction with said immobilized barcode templates and transposase.

Another embodiment of the present disclosure is a method of generating a library of barcode tagged DNA fragments comprising providing said clonally barcode tagging nucleic acid targets on a solid support; after heating, protease or a protein denaturing agent treatment, the immobilized barcode tagged fragments is treated with a DNA polymerase to fill in the gaps created in the transposition reaction; releasing barcode tagged DNA fragments with a primer extension reaction. In some embodiments, the primer has nucleotide sequence same as a portion of or the whole transposon joining strand sequence in said transpososome. The released barcode tagged fragments are sequencing ready library when sequencing library adapter sequences are included in the said primer sequence and said barcode template sequence. The released barcode tagged fragments are further amplified with primers containing library adapter sequences to generate sequencing ready library. The library contains sample specific index introduced in said primer extension reaction or said amplification reaction; therefore, libraries from different samples can be pooled together for sequencing. The sequencing reads of said barcode tagged nucleic acid fragments are grouped into a string of tandem reads from the same nucleic acid targets; which are capable for haplotype phasing. Cleavage reaction to release the immobilized barcode templates from the solid support is another embodiment.

Another embodiment of the present disclosure is a method of generating a library of targeted gene, genes or exome with barcode tagged nucleic acid fragments comprising providing said released barcode tagged nucleic acid fragments; performing primer extension reaction with first set of primers for targeted gene, genes or exome; and performing amplification reaction with a common primer containing a portion of said barcode template sequence and a second set of primers for target gene, genes, or exome; wherein said second set of primers are nested in the product of said first set of primers. The adapter sequence for sequencing library is added during the amplification step.

Another embodiment of the present disclosure is a method of generating a library of targeted gene, genes or exome with barcode tagged nucleic acid fragments comprising providing said released barcode tagged nucleic acid fragments; performing amplification with a common primer containing a portion of said barcode template sequence and first set of primers for targeted gene, genes or exome; performing amplification with a common primer containing a portion of said barcode template sequence and a second set of primers for target gene, genes, or exome; and the second set of primers are nested in the product of said first set of primers. The adapter sequence for sequencing library is added during the amplification step.

Another embodiment of the present disclosure is a method of generating a library of targeted gene, genes or exome with barcode tagged nucleic acid fragments comprising providing said clonally barcode tagging nuclei acid targets on a solid support; performing primer extension reaction with first set of primers for targeted gene, genes or exome; performing amplification with a common primer containing a portion of said barcode template sequence and a second set of primers for target gene, genes, or exome; and the second set of primers are nested in the product of said first set of primers. The adapter sequence for sequencing library is added during the amplification step.

Another embodiment of the present disclosure is a method of generating a library of targeted gene, genes or exome with barcode tagged nucleic acid fragments comprising providing said clonally barcode tagging nuclei acid targets on a solid support; performing amplification with a common primer containing a portion of said barcode template sequence and first set of primers for targeted gene, genes or exome; performing amplification with a common primer containing a portion of said barcode template sequence and a second set of primers for target gene, genes, or exome; and the second set of primers are nested in the product of the first set of primers. The adapter sequence for sequencing library is added during said amplification step. In some embodiments, the library contains sample specific index introduced in the prime extension reaction or said amplification reaction; therefore, libraries from different samples can be pooled together for sequencing.

Another embodiment of the present disclosure is a method of clonally barcode tagging nucleic acid targets comprising providing beads or microparticles having clonal barcode templates immobilized thereupon; providing a transposable DNA; applying nucleic acid targets to said transposable DNA and transposase to form DNA-transpososome strings in solution; encapsulating said DNA-transpososome strings, the beads or microparticles having barcode templates, and aqueous reaction reagents into water-in-oil emulsion droplets; applying a heat treatment to release said transposase from said transpososomes to break said nucleic acid target into fragments in the emulsion droplets; and driving the nucleic acid fragments onto the said barcode templates on said beads or microparticles.

Another embodiment of the present disclosure is a method of clonally barcode tagging nucleic acid targets comprising providing a transposable DNA; applying nucleic acid targets to said transposable DNA and transposase to form nucleic acid-transpososome complexes in solution; encapsulating said nucleic acid-transpososome complexes and aqueous reaction reagents into water-in-oil emulsion droplets as target droplets; providing clonal barcode templates in water-in-oil droplets as barcode droplets; merging the target droplets with barcode droplets one by one; applying a heat treatment to said merged droplets to release said transposase from said transpososomes to break said DNA target into fragments inside the emulsion droplets; and attaching the said barcode to said DNA fragments in the droplets.

Although the invention has been explained with respect to an embodiment, it is to be understood that many other possible modifications and variations can be made without departing from the spirit and scope of the invention as herein described.

Further, in general with regard to the processes, systems, methods, etc. described herein, it should be understood that, although the steps of such processes, etc. have been described as occurring according to a certain ordered sequence, such processes could be practiced with the described steps performed in an order other than the order described herein. It further should be understood that certain steps could be performed simultaneously, that other steps could be added, or that certain steps described herein could be omitted. In other words, the descriptions of processes herein are provided for the purpose of illustrating certain embodiments, and should in no way be construed so as to limit the claimed invention.

Moreover, it is to be understood that the above description is intended to be illustrative and not restrictive. Many embodiments and applications other than the examples provided would be apparent to those of skill in the art upon reading the above description. The scope of the invention should be determined, not with reference to the above description, but should instead be determined with reference to the appended claims, along with the full scope of equivalents to which such claims are entitled. It is anticipated and intended that future developments will occur in the arts discussed herein, and that the disclosed systems and methods will be incorporated into such future embodiments. In sum, it should be understood that the invention is capable of modification and variation and is limited only by the following claims.

Lastly, all defined terms used in the application are intended to be given their broadest reasonable constructions consistent with the definitions provided herein. All undefined terms used in the claims are intended to be given their broadest reasonable constructions consistent with their ordinary meanings as understood by those skilled in the art unless an explicit indication to the contrary is made herein. In particular, use of the singular articles such as "a," "the," "said," etc. should be read to recite one or more of the indicated elements unless a claim recites an explicit limitation to the contrary.

### Example 1

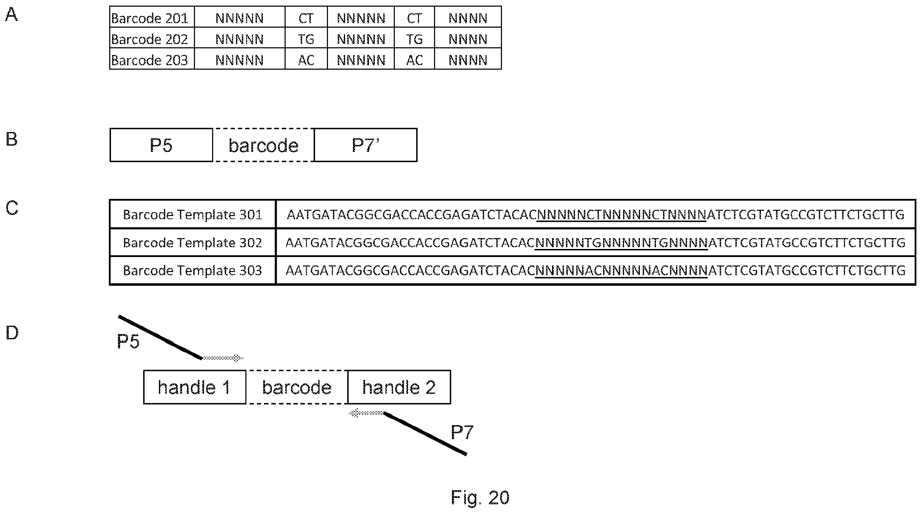

This example describes a specific barcode design based on the concept described in Fig. 1. Barcode 201 in Fig. 20A has five degenerate bases, followed by C and T, followed by another five degenerate bases, followed by C and T, and four degenerate bases at the end. Barcode 202 and Barcode 203 sequences are very similar as Barcode 201 except the known sequences are TG and AC, respectively. These barcodes can be flanked by P5 and P7' adapter sequences used by Illumina sequencing platform to form barcode templates (Fig. 20B). Fig. 20C shows the detail sequences of barcode templates in the format as Fig. 20B. In some cases, sequencing platform specific adapter (e.g. P5 and P7 sequences for Illumina sequencing platform) can be introduced at a later stage as Fig. 20D. The barcode template design in Fig. 20D has a platform independent universal handle 1 and handle 2. Illumina platform specific P5 and P7 adapter sequences may be added by PCR amplification to generate sequencing library for sequencing detection.

P7 oligonucleotide (5'-CAAGCAGAAGACGGCATACGAGAT-3') was synthesized with an amine group at the 5' end and a six-carbon linker (C6) between the amine and the other nucleotides (integrated DNA Technologies, Coralville, IA). This oligonucleotide was conjugated to Dynabeads^{®} M-270 Carboxylic Acid beads per manufacturer's protocol. Barcode templates 301, 302 and 303 were synthesized separately and pooled in equal molarity. They were clonally amplified to beads conjugated with P7 oligonucleotides according to the BEAMing protocol (Diehl et al, 2005) using P7 as forward primer and P5 (5'-AATGATACGGCGACCACCGAGATCTACAC-3') as reverse primer. Clonally amplified beads were collected. Barcode templates on the beads were further amplified off the beads using P5 and P7 primers and sequenced on a MiniSeq instrument to evaluate the system performance.

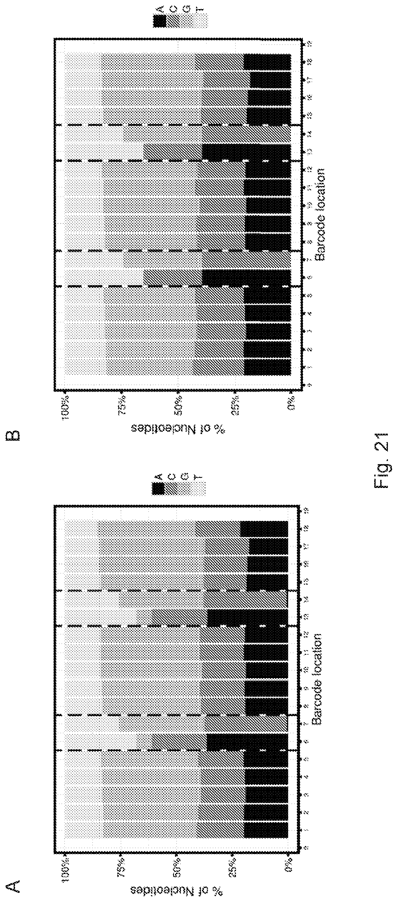

Fig. 21A showed nucleotide content at each position of all the sequenced barcode reads. G base content was between 42% to 47% at all degenerate base locations instead of 25% expected if distribution of A, C, G and T in the degenerate positions was truly random during oligonucleotides synthesis. Degenerate nucleotides were synthesized via machine mix method, which is known to create certain biased representation among the four nucleotides, especially G over-representation. However, G base content in the sequenced barcode appeared much higher than expected G bias. Barcode positions 6 and 13 should have no any Gs; barcode positions 7 and 14 should have no any As. The sequencing data showed approximately 6.7% G each at positions 6 and 13, and approximately 0.7% A each at positions 7 and 14 (Fig. 21A). These G and A may be partially due to oligonucleotides synthesis error. But we don't expect much higher G mis-incorporation rate during synthesis than that of A mis-incorporation. The likely source of more overcalled G (approximately 6%) was from sequencing related error. Fig. 21B showed nucleotide content for only barcodes with correct base at positions 6, 7, 13, and 14 when all the barcode reads showed any wrong sequence at positions 6, 7, 13, and 14 were filtered out. G base content decreased and was between 38% to 44% at all degenerate base locations. This suggested that filtered out barcode reads had much higher G representation across all degenerate positions, which were likely error prone reads generated by sequencing error.

### Example 2

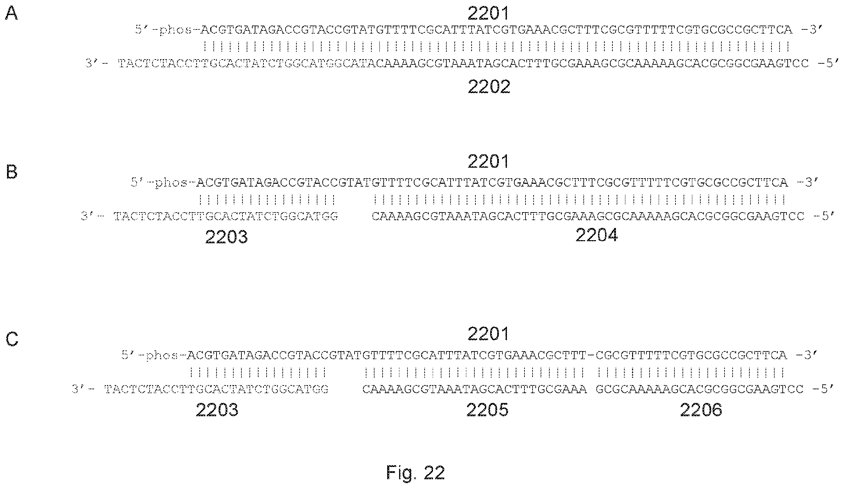

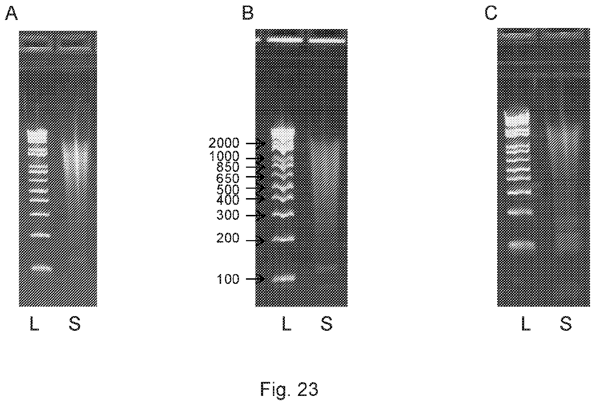

This example describes specifically designed MuA transposable DNAs and its transposition functionality with a C-terminal His-tagged MuA transposase. One of MuA transposable DNA designs (Fig. 22 A) has joining strand (2201) and complementary strand (2202) each in a single piece. The complementary strand has a 3' end overhang, which can be used as a linker for capture by barcode templates on an immobilized solid support. Second design (Fig. 22B) has two pieces for the complementary strand (2203 and 2204), which will improve the flexibility of the overhanging tail for capture. Third design (Fig. 22C) has 3 pieces for the complementary strand (2203, 2205, 2206), which will simplify the oligonucleotides synthesis. A C-terminal 6x His-tag MuA transposase was expressed in *E. coli* BL21 and purified to homogeneity. 1ng E. *coli* genomic DNA was incubate with 0.05uM MuA transposable DNA and 0.3ng MuA transposase in a buffer containing Tris-HCl, pH 8.0, NaCl, MgCl₂, DMSO and PEG-8000 at 37°C for 30 minutes to 60 minutes. After the incubation, SDS was added to final 0.05%. One tenth of reaction mixture was used to set up a 30ul PCR reaction with Phusion Hot Start II High Fidelity PCR master mix for 18-cycle amplification. 10uL of PCR products were loaded onto a 2% E-gel EX. DNA fragments from tagmentation ranged from 150bp to 2Kbp were clearly observed for all three designs of MuA transposable DNA (Fig. 23).

In some cases, the linker oligonucleotides (2203) may not be annealed to the transposable DNA during transposition reaction. It can be used in the capture reaction only when transposable DNA ends need to attach to barcode templates.

### Example 3

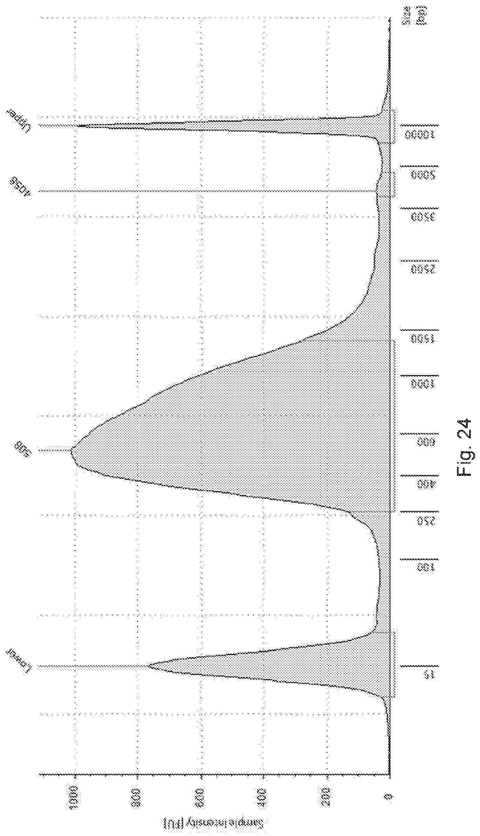

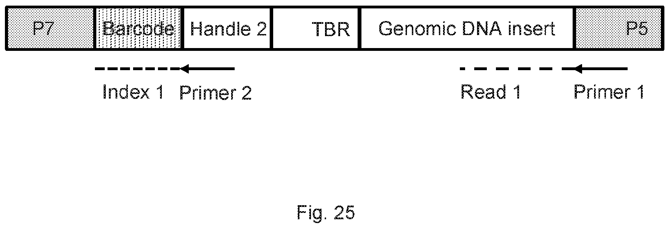

This example describes a method of barcode tagging of DNA with barcoded beads in an open bulk reaction without additional partition. Modified barcode templates 301, 302 and 303 using a different universal sequence for handle 1 were pooled in equal molarity, and clonally amplified to beads conjugated with P7 oligonucleotides according to the BEAMing protocol (Diehl et al, 2005). Beads with single stranded DNA were collected directly after BEAMing reaction as barcoded beads. 1ng *E. coli* genomic DNA was tagmented by incubating with 0.05uM MuA transposable DNA as design B in Fig. 22 and 0.3ng MuA transposase in a buffer containing Tris-HCl, pH 8.0, NaCl, MgCl₂, DMSO and PEG-8000 at 37°C for 30 minutes. Tagmentation reaction mixture was then incubated with 20 million barcoded beads in a Rapid Ligation Buffer using T4 DNA ligase (Enzymatics, Beverly, MA) for 15 minutes at 37°C to capture the tagmented DNA on to the barcoded beads. EDTA was added to inactivate the capture reaction. Reaction mixture was heated at 72°C for 5 minutes to release transposase from tagged DNA. Washed beads were treated with exonuclease I to remove single stranded polynucleotides, and then used for 20 cycle PCR amplification to release immobilized barcode tagged DNA fragments. PCR products was purified with 0.8X AMPure XP beads to remove small primer dimers and examined using a high sensitivity D5000 screentape on a TapeStation (Fig. 24). The purified PCR products are used as an Illumina sequencing library (Fig. 25), which can be sequenced to determine both genomic DNA insert sequence using sequencing primer 1 and barcode sequence using sequencing primer 2.

### Example 4

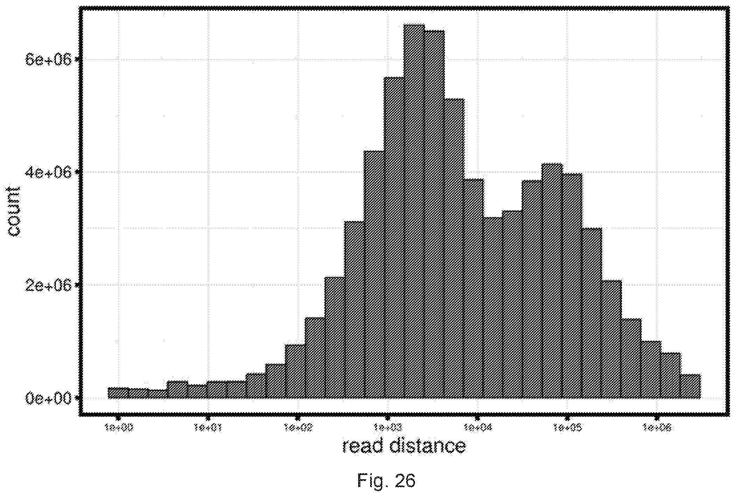

This example demonstrates the contiguity of barcode tagged DNA sequencing reads. A barcode tagged *E. coli* DNA library described in Example 3 was sequenced on a NextSeq 500 instrument with 73-cycle Read 1 sequencing for genomic DNA insert and 18-cycle Index 1 sequencing for barcode sequences (Fig. 25). The output bcl file were converted to fastq file, which was used for further analyses. Based on the known nucleotide sequences in the barcode templates 301, 302 and 303, approximately 10% barcode reads contained more than one error among the four positions, which were excluded from downstream data analysis. Read 1 reads with the same barcode sequence were sorted for each barcode based on the reference genome alignment location. Read distance to the next alignment was calculated and read count frequency along the read distance was plotted in Fig. 26. When barcoded reads kept the contiguity of the original tagged DNA fragment, piling up of proximal reads was expected. The read distance from the original DNA fragment before tagging would also pile up as reads with longer distance. A bi-modal distribution of read count frequency plot would be expected, which was exactly observed in the Fig. 26. Strong enrichment of shorter distance reads with a peak around 3Kb demonstrated successful barcode reads contiguity.

### REFERENCES

Adey A et al. 2010. Genome Biol. 11, R119.
Amini S et al. 2014. Nature Genetics, 46(12):1343-1349.
Au T et al. 2004. EMBO J., 23: 3408-3420.
Boeke J. D. 1989. Transposable elements in Saccharomyces cerevisiae in Mobile DNA. pp. 335-374 in Mobile DNA, edited by D. E. BERG and. M. M. HOWE.
Burton B.M. and Baker T.A. 2003. Chemistry & Biology 10: 463-472.
Caruccio, N. 2011. Methods Mol. Biol. 733: 241-255.
Craig N. L. 1996. Transposon Tn7. Curr. Top. Microbiol. Immunol. 204: 27-48.
Devine, S. E. and Boeke, J. D. 1994. Nucleic Acids Research, 22(18): 3765-3772.
Diehl F. et al. 2005. PNAS, 102 (45): 16368-16373.
Haapa, S. et al. 1999. Nucleic Acids Research, 27(13): 2777-2784.
Ichikawa H. and Ohtsubo E. 1990. J. Biol. Chem., 265(31): 18829-32.
Kaufman P. and Rio D. C. 1992. Cell, 69(1): 27-39.
Kavanagh I, Kiiskinen L. L. and Haakana H. 2013. Unite State Patent Application Publication US2013/0023423.
Kleckner N. et al. 1996. Curr. Top. Microbiol. Immunol., 204: 49-82.
Macosko et al., 2015. Cell, 161: 1202-1214.
Mizuuchi M., Baker T.A. and Mizuuchi K. 1992. Cell 70, 303-311.
Lampe D. J., Churchill M. E. A. and Robertson H. M. 1996. EMBO J., 15(19): 5470-5479.
Ohtsubo E. & Sekine Y. 1996. Curr. Top. Microbiol. Immunol., 204:126.
Park B. T., Jeong M. H. and Kim B. H. 1992. Taehan Misaengmul Hakhoechi, 27(4): 381-9.
Savilahti H., P. A. Rice, and K. MiZuuchi. 1995. EMBO J. 14:4893-4903.
Surette M., Buch S.J. and Chaconas G. 1987. Cell 70: 303-311.
Varmus H. and Brown. P. A. 1989. Retroviruses, in Mobile DNA. Berg D. E. and Howe M. eds. American Society for Microbiology, Washington D. C. pp. 53-108.
Vos J. C., Baere I. and Plasterk R. H. A. 1996. Genes Dev., 10(6): 755-61.

## Claims

1. A method for tracking nucleic acid target origin by barcode tagging comprising:
a. providing, in an open bulk reaction without partition, a plurality of microparticles, wherein some microparticles each have a clonal barcode template or a semi clonal barcode template immobilized thereon, wherein each barcode template comprises a barcode sequence and at least one flanking handle sequence; wherein other microparticles do not have barcode templates and serve as spacer in downstream reactions;
b. providing a transposable DNA and a transposase;
c. contacting a nucleic acid target with said transposase and said transposable DNA to form a transpososome-nucleic acid complex;
d. attaching the nucleic acid target within said transpososome-nucleic acid complex to a barcode template on a microparticle;
wherein the attached transpososome-nucleic acid complex is not divided from a second transpososome-nucleic acid complex by a partition; and
e. breaking said nucleic acid target into fragments by removing said transposase from said transpososome-nucleic acid complex, wherein each fragment of the target nucleic acid target is attached to a barcode template, thereby forming barcode tagged fragments.

2. The method for tracking nucleic acid target origin by barcode tagging of claim 1,
wherein providing the microparticles further comprises immobilizing a barcode template on a microparticle by a clonal amplification method; and
wherein after the clonal amplification method, the microparticle that has the amplified barcode template thereon is not separated from the microparticle that does not have the amplified barcode template thereon; and/or
further comprising adding additional microparticle that does not have the amplified barcode template thereon.

3. The method for tracking nucleic acid target origin by barcode tagging of claim 1 or claim 2,
wherein said transposable DNA has no complementary sequence to said barcode template;
wherein attaching said transpososome-nucleic acid complex to said microparticle further comprises using a linker oligo
wherein each linker oligo has a complementary sequence of said barcode template at one end and a complementary sequence of said transposable DNA at another end; and wherein contacting the nucleic acid with the transposase and the transposable DNA forms a plurality of transpososome-nucleic acid complexes.

4. The method for tracking nucleic acid target origin by barcode tagging of claim 1, claim 2 or claim 3 further comprising:
a. denaturing the barcode tagged fragments, thereby producing a plurality of single stranded barcode tagged fragments immobilized on the microparticles; and
b. releasing the single stranded barcode tagged nucleic acid fragments from the microparticles and/or copying the single stranded barcode tagged nucleic acid fragments through primer extension or amplification to generate a soluble library.

5. The method for tracking nucleic acid target origin by barcode tagging of any one of claims 1 to 4 further comprising:
repairing by a polymerase any gap produced during the contacting step (c).

6. The method for tracking nucleic acid target origin by barcode tagging of any one of claims 1 to 5,
wherein a plurality of the barcode templates is clonally or semi-clonally immobilized onto the entire surface of the microparticles.

7. The method for tracking nucleic acid target origin by barcode tagging of any one of claims 1 to 6, wherein said transposase is selected from the group consisting of Tn, Mu, Ty, and Tc transposases in a wildtype or a mutant or a tagged version thereof, and a combination thereof.

8. The method for tracking nucleic acid target origin by barcode tagging of any one of claims 1 to 7, wherein the transposase is a MuA transposase, or a Tn5 transposase, or a combination thereof.

9. The method for tracking nucleic acid target origin by barcode tagging of any one of claims 1 to 8, wherein said transposable DNA comprises a transposon, wherein the transposon is selected from the group consisting of Tn, Mu, Ty, and Tc transposon DNAs in a wildtype or a mutant version thereof, and a combination thereof.

10. The method for tracking nucleic acid target origin by barcode tagging of any one of claims 1 to 9, wherein said transposon is a Tn5 transposon, or a MuA transposon, or a combination thereof.

11. The method for tracking nucleic acid target origin by barcode tagging of any one of claims 1 to 10, wherein attaching the nucleic acid target within the transpososome-nucleic acid complex to the barcode template is by ligation, hybridization or a combination thereof.

12. The method of claim 5 further comprising:
generating a soluble library by releasing a non-immobilized complementary strand of the repaired double stranded barcode tagged nucleic acid target fragment from the microparticles, and/or
releasing the double stranded repaired barcode tagged nucleic acid target fragment from the microparticles, and/or
copying the barcode tagged fragments on the microparticles through primer extension or amplification.

13. The method for tracking nucleic acid target origin by barcode tagging of any one of claims 1 to 12, wherein the attaching step (d) further comprises adding a linker oligo wherein said oligo linker is single stranded or partially single stranded.

14. The method for tracking nucleic acid target origin by barcode tagging of any one of claims 1 to 13, wherein said breaking said nucleic acid target into fragments further comprises treating said nucleic acid target by heating, by degradation with a protease, by denaturation with a protein denaturing agent, or a combination thereof.

15. The method of claim 4 or claim 12, wherein said primer extension or amplification utilizes a first set of primers selected from the group consisting of random primers primers for common adaptors, first set of gene specific primers, first set of exome specific primers.

16. The method of claim 12, or any one of claims 13 to 15 when dependent on claim 12, wherein the said soluble library is used to determine phasing information of the nucleic acid target.

17. The method of 12, or any one of claims 13 to 16 when dependent on claim 12, wherein the said soluble library is amplified with a first set primer containing a portion of said barcode template sequence and a second set of gene specific primers or exome specific primers; wherein said second set of primers are nested in the product of said first set of primers.

18. The method of any one of claims 1 to 17, wherein said microparticles having clonal barcode templates or semi clonal barcode templates immobilized thereon are produced by methods of direct synthesis, clonal amplification, or a combination thereof.

19. The method of claim 2, or any one of claims 3 and 18 when dependent on claim 2, wherein said clonal amplification is selected from the group consisting of emulsion PCR, bridge PCR, isothermal amplification, template walking.

20. The method of any one of claims 1 to 19, wherein said barcode template comprises a barcode comprising a nucleic acid sequence with a length between 4 to 100 bases and configured to limit homopolymer length and sequencing errors.

21. The method of claim 20, wherein said barcode further comprises:
at least two random degenerate segments each being at least 2 nucleotide bases in length and at least one non-homopolymer segment each being at least 2 non-homopolymer bases in length,
wherein the random degenerate segments and non-homopolymer segments are arranged alternatively one after another, with the non-homopolymer segments serving as homopolymer breakers of the random segments and/or nucleic acid sample identification markers, and
wherein the random segment at any position can be any one of 2, 3 or 4 nucleotides chosen from A, C, G, and T/U and a modified version of the nucleotide thereof; wherein said non-homopolymer segments have a different nucleotide base at the first and the last position.

22. The method of claim 21, wherein said barcode is flanked by a handle sequence at each end, wherein said handle sequence is used as a binding site for amplification, hybridization, annealing, and/or ligation.

## Patentansprüche

1. Verfahren zum Verfolgen des Ursprungs eines Nukleinsäureziels durch Barcode-Kennzeichnung, umfassend:
a. Bereitstellen einer Vielzahl von Mikropartikeln in einer offenen Massenreaktion ohne Partition, wobei einige Mikropartikel jeweils eine darauf immobilisierte klonale Barcode-Vorlage oder eine semiklonale Barcode-Vorlage aufweisen, wobei jede Barcode-Vorlage eine Barcode-Sequenz und mindestens eine flankierende Handhabungssequenz umfasst; wobei andere Mikropartikel keine Barcode-Vorlagen aufweisen und in nachgeschalteten Reaktionen als Abstandshalter dienen;
b. Bereitstellen einer transponierbaren DNA und einer Transposase;
c. Inkontaktbringen eines Nukleinsäureziels mit der Transposase und der transponierbaren DNA, um einen Transpososom-Nukleinsäure-Komplex zu bilden;
d. Anbringen des Nukleinsäureziels innerhalb des Transpososom-Nukleinsäure-Komplexes an eine Barcode-Vorlage auf einem Mikropartikel;
wobei der angebrachte Transpososom-Nukleinsäure-Komplex nicht durch eine Partition von einem zweiten Transpososom-Nukleinsäure-Komplex getrennt ist; und
e. Aufbrechen des Nukleinsäureziels in Fragmente durch Entfernen der Transposase aus dem Transpososom-Nukleinsäure-Komplex, wobei jedes Fragment des Ziel-Nukleinsäureziels an einer Barcode-Vorlage angebracht wird, wodurch mit einem Barcode gekennzeichnete Fragmente gebildet werden.

2. Verfahren zum Verfolgen des Ursprungs eines Nukleinsäureziels durch Barcode-Kennzeichnung nach Anspruch 1, wobei das Bereitstellen der Mikropartikel weiter das Immobilisieren einer Barcode-Vorlage auf einem Mikropartikel durch ein klonales Amplifikationsverfahren umfasst; und
wobei das Mikropartikel, das darauf die amplifizierte Barcode-Vorlage aufweist, nach dem klonalen Amplifikationsverfahren nicht von dem Mikropartikel getrennt wird, das nicht die amplifizierte Barcode-Vorlage darauf aufweist; und/oder
weiter umfassend das Hinzufügen eines zusätzlichen Mikropartikels, das nicht die amplifizierte Barcode-Vorlage darauf aufweist.

3. Verfahren zum Verfolgen des Ursprungs eines Nukleinsäureziels durch Barcode-Kennzeichnung nach Anspruch 1 oder Anspruch 2,
wobei die transponierbare DNA keine komplementäre Sequenz zur Barcode-Vorlage aufweist;
wobei das Anbringen des Transpososom-Nukleinsäure-Komplexes an das Mikropartikel weiter das Verwenden eines Linker-Oligos umfasst, wobei jedes Linker-Oligo an einem Ende eine komplementäre Sequenz der Barcode-Vorlage und an einem anderen Ende eine komplementäre Sequenz der transponierbaren DNA aufweist; und wobei das Inkontaktbringen der Nukleinsäure mit der Transposase und der transponierbaren DNA eine Vielzahl von Transpososom-Nukleinsäure-Komplexen bildet.

4. Verfahren zum Verfolgen des Ursprungs eines Nukleinsäureziels durch Barcode-Kennzeichnung nach Anspruch 1, Anspruch 2 oder Anspruch 3, das weiter umfasst:
a. Denaturieren der mit Barcode gekennzeichneten Fragmente, wodurch eine Vielzahl einzelsträngiger, mit Barcode gekennzeichneter Fragmente erzeugt wird, die auf den Mikropartikeln immobilisiert sind; und
b. Freisetzen der einzelsträngigen, mit Barcode gekennzeichneten Nukleinsäurefragmente aus den Mikropartikeln und/oder Kopieren der einzelsträngigen, mit Barcode gekennzeichneten Nukleinsäurefragmente durch Primerverlängerung oder - amplifikation, um eine lösliche Bibliothek zu erzeugen.

5. Verfahren zum Verfolgen des Ursprungs eines Nukleinsäureziels durch Barcode-Kennzeichnung nach einem der Ansprüche 1 bis 4, das weiter umfasst:
Reparieren jeder Lücke, die während des Schrittes des Inkontaktbringens (c) entstanden ist, durch eine Polymerase.

6. Verfahren zum Verfolgen des Ursprungs eines Nukleinsäureziels durch Barcode-Kennzeichnung nach einem der Ansprüche 1 bis 5,
wobei eine Vielzahl der Barcode-Vorlagen klonal oder semiklonal auf der gesamten Oberfläche der Mikropartikel immobilisiert ist.

7. Verfahren zum Verfolgen des Ursprungs eines Nukleinsäureziels durch Barcode-Kennzeichnung nach einem der Ansprüche 1 bis 6, wobei die Transposase aus der Gruppe ausgewählt ist, bestehend aus Tn-, Mu-, Ty- und Tc-Transposasen in einem Wildtyp oder einem Mutanten oder einer gekennzeichneten Version davon, und einer Kombination davon.

8. Verfahren zum Verfolgen des Ursprungs eines Nukleinsäureziels durch Barcode-Kennzeichnung nach einem der Ansprüche 1 bis 7, wobei die Transposase eine MuA-Transposase oder eine Tn5-Transposase oder eine Kombination davon ist.

9. Verfahren zum Verfolgen des Ursprungs eines Nukleinsäureziels durch Barcode-Kennzeichnung nach einem der Ansprüche 1 bis 8, wobei die transponierbare DNA ein Transposon umfasst, wobei das Transposon aus der Gruppe ausgewählt ist, bestehend aus Tn-, Mu-, Ty- und Tc-Transposon-DNAs in einem Wildtyp oder einer mutierten Version davon, und einer Kombination davon.

10. Verfahren zum Verfolgen des Ursprungs eines Nukleinsäureziels durch Barcode-Kennzeichnung nach einem der Ansprüche 1 bis 9, wobei das Transposon ein Tn5-Transposon oder ein MuA-Transposon oder eine Kombination davon ist.

11. Verfahren zum Verfolgen des Ursprungs eines Nukleinsäureziels durch Barcode-Kennzeichnung nach einem der Ansprüche 1 bis 10, wobei das Anbringen des Nukleinsäureziels innerhalb des Transpososom-Nukleinsäure-Komplexes an die Barcode-Vorlage durch Ligation, Hybridisierung oder eine Kombination davon erfolgt.

12. Verfahren nach Anspruch 5, weiter umfassend:
Erzeugen einer löslichen Bibliothek durch Freisetzen eines nicht immobilisierten komplementären Strangs des reparierten doppelsträngigen, mit Barcode gekennzeichneten Nukleinsäure-Zielfragments aus den Mikropartikeln, und/oder
Freisetzen des doppelsträngigen, reparierten, mit Barcode gekennzeichneten Nukleinsäure-Zielfragments aus den Mikropartikeln, und/oder
Kopieren der mit Barcode gekennzeichneten Fragmente auf den Mikropartikeln durch Primerverlängerung oder -amplifikation.

13. Verfahren zum Verfolgen des Ursprungs eines Nukleinsäureziels durch Barcode-Kennzeichnung nach einem der Ansprüche 1 bis 12, wobei der Schritt des Anbringens (d) weiter das Hinzufügen eines Linker-Oligos umfasst, wobei der Oligo-Linker einzelsträngig oder teilweise einzelsträngig ist.

14. Verfahren zum Verfolgen des Ursprungs eines Nukleinsäureziels durch Barcode-Kennzeichnung nach einem der Ansprüche 1 bis 13, wobei das Aufbrechen des Nukleinsäureziels in Fragmente weiter das Behandeln des Nukleinsäureziels durch Erhitzen, durch Abbau mit einer Protease, durch Denaturierung mit einem Protein-Denaturierungsmittel oder eine Kombination davon umfasst.

15. Verfahren nach Anspruch 4 oder Anspruch 12, wobei die Primerverlängerung oder -amplifikation einen ersten Satz von Primern verwendet, ausgewählt aus der Gruppe bestehend aus Zufallsprimern, Primern für gemeinsame Adaptoren, einem ersten Satz genspezifischer Primer, einem ersten Satz exomspezifischer Primer.

16. Verfahren nach Anspruch 12 oder einem der Ansprüche 13 bis 15, wenn von Anspruch 12 abhängig, wobei die lösliche Bibliothek zum Bestimmen von Phaseninformationen des Nukleinsäureziels verwendet wird.

17. Verfahren nach Anspruch 12 oder einem der Ansprüche 13 bis 16, wenn von Anspruch 12 abhängig, wobei die lösliche Bibliothek mit einem ersten Primer-Satz, der einen Abschnitt der Barcode-Vorlagesequenz enthält, und einem zweiten Satz an genspezifischen oder exomspezifischen Primern amplifiziert wird; wobei der zweite Satz an Primern in das Produkt des ersten Satzes an Primern eingebettet ist.

18. Verfahren nach einem der Ansprüche 1 bis 17, wobei die Mikropartikel, die darauf immobilisierte klonale Barcode-Vorlagen oder semiklonale Barcode-Vorlagen aufweisen, durch Verfahren direkter Synthese, klonaler Amplifikation oder einer Kombination davon hergestellt werden.

19. Verfahren nach Anspruch 2 oder einem der Ansprüche 3 und 18, wenn von Anspruch 2 abhängig, wobei die klonale Amplifikation aus der Gruppe ausgewählt ist, bestehend aus Emulsions-PCR, Brücken-PCR, isothermer Amplifikation und Vorlagen-Walking.

20. Verfahren nach einem der Ansprüche 1 bis 19, wobei die Barcode-Vorlage einen Barcode umfasst, der eine Nukleinsäuresequenz mit einer Länge zwischen 4 und 100 Basen umfasst und konfiguriert ist, um Homopolymerlänge und Sequenzierungsfehler zu begrenzen.

21. Verfahren nach Anspruch 20, wobei der Barcode weiter umfasst:
mindestens zwei zufällige degenerierte Segmente, von denen jedes mindestens 2 Nukleotidbasen lang ist, und mindestens ein Nicht-Homopolymer-Segment, von denen jedes mindestens 2 Nicht-Homopolymer-Basen lang ist,
wobei die zufälligen degenerierten Segmente und Nicht-Homopolymer-Segmente abwechselnd nacheinander angeordnet sind, wobei die Nicht-Homopolymer-Segmente als Homopolymer-Brecher der zufälligen Segmente und/oder Marker zur Identifizierung von Nukleinsäureproben dienen, und
wobei das zufällige Segment an jeder Position eines von 2, 3 oder 4 Nukleotiden, ausgewählt aus A, C, G und T/U und eine modifizierte Version des Nukleotids davon sein kann; wobei die Nicht-Homopolymer-Segmente an der ersten und der letzten Position eine unterschiedliche Nukleotidbase aufweisen.

22. Verfahren nach Anspruch 21, wobei der Barcode an jedem Ende von einer Handhabungssequenz flankiert wird, wobei die Handhabungssequenz als Bindungsstelle zur Amplifikation, Hybridisierung, Vergütung und/oder Ligation verwendet wird.

## Revendications

1. Procédé pour suivre l'origine d'une cible d'acide nucléique par marquage par code à barres comprenant :
a. la fourniture, dans une réaction en masse ouverte sans séparation, d'une pluralité de microparticules, dans lequel certaines microparticules présentent chacune un modèle de code à barres clonal ou un modèle de code à barres semi-clonal immobilisé sur celles-ci, dans lequel chaque modèle de code à barres comprend une séquence de codes à barres et au moins une séquence poignées flanquantes ; dans lequel d'autres microparticules ne présentent pas de modèles de codes à barres et servent d'espaceur dans les réactions en aval ;
b. la fourniture d'un ADN transposable et d'une transposase ;
c. la mise en contact d'une cible d'acide nucléique avec ladite transposase et ledit ADN transposable pour former un complexe transpososome-acide nucléique ;
d. la fixation de la cible d'acide nucléique à l'intérieur dudit complexe transpososome-acide nucléique à un modèle de code à barres sur une microparticule ; dans lequel le complexe transpososome-acide nucléique fixé n'est pas séparé d'un second complexe transpososome-acide nucléique par une séparation ; et
e. la coupure de ladite cible d'acide nucléique en fragments en retirant ladite transposase dudit complexe transpososome-acide nucléique, dans lequel chaque fragment de la cible d'acide nucléique cible étant fixé à un modèle de code à barres, formant ainsi des fragments marqués par un code à barres.

2. Procédé pour suivre l'origine d'une cible d'acide nucléique par marquage par code à barres selon la revendication 1, dans lequel la fourniture des microparticules comprend en outre l'immobilisation d'un modèle de code à barres sur une microparticule par un procédé d'amplification clonale ; et
dans lequel après le procédé d'amplification clonale, la microparticule qui présente le modèle de code à barres amplifié n'est pas séparée de la microparticule qui ne présente pas le modèle de code à barres amplifié ; et/ou
comprenant en outre l'ajout d'une microparticule supplémentaire sur laquelle ne figure pas le modèle de code à barres amplifié.

3. Procédé pour suivre l'origine d'une cible d'acide nucléique par marquage par code à barres selon la revendication 1 ou la revendication 2,
dans lequel ledit ADN transposable ne présente pas de séquence complémentaire sur ledit modèle de code à barres ;
dans lequel la fixation dudit complexe transpososome-acide nucléique à ladite microparticule comprend en outre l'utilisation d'un oligo-lieur, dans lequel chaque oligo-lieur présente une séquence complémentaire dudit modèle de code à barres à une extrémité et une séquence complémentaire dudit ADN transposable à une autre extrémité ; et dans lequel la mise en contact de l'acide nucléique avec la transposase et l'ADN transposable forme une pluralité de complexes transpososome-acide nucléique.

4. Procédé pour suivre l'origine d'une cible d'acide nucléique par marquage par code à barres selon la revendication 1, la revendication 2 ou la revendication 3, comprenant en outre :
a. la dénaturation des fragments marqués par un code à barres, produisant ainsi une pluralité de fragments marqués par un code à barres simple brin immobilisés sur les microparticules ; et
b. la libération des fragments d'acide nucléique marqués par un code à barres simple brin des microparticules et/ou la copie des fragments d'acide nucléique marqués par un code à barres simple brin par extension ou amplification d'amorce pour générer une bibliothèque soluble.

5. Procédé pour suivre l'origine d'une cible d'acide nucléique par marquage par code à barres selon l'une quelconque des revendications 1 à 4, comprenant en outre :
la réparation par une polymérase de tout espace produit lors de l'étape de mise en contact (c).

6. Procédé pour suivre l'origine d'une cible d'acide nucléique par marquage par code à barres selon l'une quelconque des revendications 1 à 5,
dans lequel une pluralité de modèles de codes à barres sont immobilisés de manière clonale ou semi-clonale sur la totalité de la surface des microparticules.

7. Procédé pour suivre l'origine d'une cible d'acide nucléique par marquage par code à barres selon l'une quelconque des revendications 1 à 6, dans lequel ladite transposase est choisie parmi le groupe consistant en transposases Tn, Mu, Ty et Tc dans un type sauvage ou un mutant ou une version marquée de celui-ci, et une combinaison de ceux-ci.

8. Procédé pour suivre l'origine d'une cible d'acide nucléique par marquage par code à barres selon l'une quelconque des revendications 1 à 7, dans lequel la transposase est une transposase MuA, ou une transposase Tn5, ou une combinaison de celles-ci.

9. Procédé pour suivre l'origine d'une cible d'acide nucléique par marquage par code à barres selon l'une quelconque des revendications 1 à 8, dans lequel ledit ADN transposable comprend un transposon, dans lequel le transposon est choisi parmi le groupe consistant en ADN de transposon Tn, Mu, Ty et Tc dans un type sauvage ou une version mutante de celui-ci, et une combinaison de ceux-ci.

10. Procédé pour suivre l'origine d'une cible d'acide nucléique par marquage par code à barres selon l'une quelconque des revendications 1 à 9, dans lequel ledit transposon est un transposon Tn5, ou un transposon MuA, ou une combinaison de ceux-ci.

11. Procédé pour suivre l'origine d'une cible d'acide nucléique par marquage par code à barres selon l'une quelconque des revendications 1 à 10, dans lequel la fixation de la cible d'acide nucléique dans le complexe transpososome-acide nucléique au modèle de code à barres se fait par ligature, hybridation ou une combinaison de celles-ci.

12. Procédé selon la revendication 5, comprenant en outre :
la génération d'une bibliothèque soluble en libérant un brin complémentaire non immobilisé du fragment cible d'acide nucléique marqué par code à barres double brin réparé à partir des microparticules, et/ou
la libération du fragment cible d'acide nucléique marqué par un code à barres réparé double brin des microparticules, et/ou
la copie des fragments marqués d'un code à barres sur les microparticules par extension ou amplification d'amorce.

13. Procédé pour suivre l'origine d'une cible d'acide nucléique par marquage par code à barres selon l'une quelconque des revendications 1 à 12, dans lequel l'étape de fixation (d) comprend en outre l'ajout d'un oligo-lieur dans lequel ledit oligo-lieur est simple brin ou partiellement simple brin.

14. Procédé pour suivre l'origine d'une cible d'acide nucléique par marquage par code à barres selon l'une quelconque des revendications 1 à 13, dans lequel ladite coupure de ladite cible d'acide nucléique en fragments comprend en outre le traitement de ladite cible d'acide nucléique par chauffage, par dégradation avec une protéase, par dénaturation avec un agent de dénaturation des protéines, ou une combinaison de ceux-ci.

15. Procédé selon la revendication 4 ou la revendication 12, dans lequel ladite extension ou amplification d'amorce utilise un premier ensemble d'amorces choisi parmi le groupe consistant en amorces aléatoires pour adaptateurs communs, un premier ensemble d'amorces spécifiques à un gène, un premier ensemble d'amorces spécifiques à un exome.

16. Procédé selon la revendication 12, ou l'une quelconque des revendications 13 à 15 lorsqu'elles dépendent de la revendication 12, dans lequel ladite bibliothèque soluble est utilisée pour déterminer des informations du phasage de la cible d'acide nucléique.

17. Procédé selon la revendication 12, ou l'une quelconque des revendications 13 à 16 lorsqu'elle dépend de la revendication 12, dans lequel ladite bibliothèque soluble est amplifiée avec un premier ensemble d'amorces contenant une partie de ladite séquence de modèle de code à barres et un second ensemble d'amorces spécifiques à un gène ou spécifiques à un exome ; dans lequel ledit second ensemble d'amorces est imbriqué dans le produit dudit premier ensemble d'amorces.

18. Procédé selon l'une quelconque des revendications 1 à 17, dans lequel lesdites microparticules sur lesquelles sont immobilisés des modèles de codes à barres clonaux ou des modèles de codes à barres semi-clonaux sont produites par des procédés de synthèse directe, d'amplification clonale ou une combinaison de ceux-ci.

19. Procédé selon la revendication 2, ou l'une quelconque des revendications 3 et 18 lorsqu'elle dépend de la revendication 2, dans lequel ladite amplification clonale est choisie parmi le groupe consistant en une PCR en émulsion, une PCR en pont, une amplification isotherme et une marche sur modèle.

20. Procédé selon l'une quelconque des revendications 1 à 19, dans lequel ledit modèle de code à barres comprend un code à barres comprenant une séquence d'acide nucléique d'une longueur comprise entre 4 et 100 bases et configuré pour limiter la longueur de l'homopolymère et les erreurs de séquençage.

21. Procédé selon la revendication 20, dans lequel ledit code à barres comprend en outre :
au moins deux segments dégénérés aléatoires présentant chacun au moins 2 bases nucléotidiques de longueur et au moins un segment non homopolymère présentant chacun au moins 2 bases non homopolymères de longueur,
dans lequel les segments dégénérés aléatoires et les segments non homopolymères sont agencés alternativement les uns après les autres, les segments non homopolymères servant de coupeurs d'homopolymère des segments aléatoires et/ou des marqueurs d'identification d'échantillons d'acide nucléique, et
dans lequel le segment aléatoire à toute position peut être l'un quelconque de 2, 3 ou 4 nucléotides choisis parmi A, C, G et T/U et une version modifiée de son nucléotide ; dans lequel lesdits segments non homopolymères présentent une base nucléotidique différente en première et en dernière position.

22. Procédé selon la revendication 21, dans lequel ledit code à barres est flanqué d'une séquence poignée à chaque extrémité, dans lequel ladite séquence poignée est utilisée comme site de liaison pour l'amplification, l'hybridation, la recuisson et/ou la ligature.
